# EUROPEAN PATENT APPLICATION

(11) **EP 4 851 130 A2**
(43) Date of publication of application: **09.12.2026**
(21) Application number: 26205068.5
(22) Date of filing: 28.06.2022
(51) Int. Cl.: C07C 49/825

(54) **POLYCATIONIC SALTS OF PHENOLIC COMPOUNDS AND USES THEREOF**

(30) Priority: 28.06.2021 US 202163215798 P; 27.07.2021 EP 21187831
(62) Document number(s) of the earlier application(s) in accordance with Art. 76 EPC: 22743681.3 / 4 337 647
(71) Applicant: Firmenich Incorporated, Plainsboro, NJ 08536 (US)
(72) Inventor: FOTSING, Joseph R., San Diego, 92121 (US); CHEN, Qing, San Diego, 92121 (US); BANAVARA, Dattatreya, Plainsboro, 08536 (US)
(74) Representative: Strych, Sebastian

(57) **Abstract**

The present disclosure generally provides poly-alkali metal salts of flavanones, dihydrochalcones, and related polyphenolic compounds. In some aspects, the disclosure provides certain compositions that include such poly-alkali metal salts of flavanones, dihydrochalcones, and related polyphenolic compounds, such as compositions that include such poly-alkali metal salts of flavanones, dihydrochalcones, and related polyphenolic compounds and one or more sweeteners. In some other aspects, the disclosure provides methods of reducing the caloric content of a sweetened article, such as a sweetened food or beverage product.

## Description

### TECHNICAL FIELD

The present disclosure generally provides poly-alkali metal salts of flavanones, dihydrochalcones, and related polyphenolic compounds. In some aspects, the disclosure provides certain compositions that include such poly-alkali metal salts of flavanones, dihydrochalcones, and related polyphenolic compounds, such as compositions that include such poly-alkali metal salts of flavanones, dihydrochalcones, and related polyphenolic compounds and one or more sweeteners. In some other aspects, the disclosure provides methods of reducing the caloric content of a sweetened article, such as a sweetened food or beverage product.

### DESCRIPTION OF RELATED ART

The taste system provides sensory information about the chemical composition of the external world. Taste transduction is one of the more sophisticated forms of chemically triggered sensation in animals. Signaling of taste is found throughout the animal kingdom, from simple metazoans to the most complex of vertebrates. Mammals are believed to have five basic taste modalities: sweet, bitter, sour, salty, and umami.

Sweetness is the taste most commonly perceived when eating foods rich in sugars. Mammals generally perceive sweetness to be a pleasurable sensation, except in excess. Caloric sweeteners, such as sucrose and fructose, are the prototypical examples of sweet substances. Although a variety of no-calorie and low-calorie substitutes exist, these caloric sweeteners are still the predominant means by which comestible products induce the perception of sweetness upon consumption.

Metabolic disorders and related conditions, such as obesity, diabetes, and cardiovascular disease, are major public health concerns throughout the world. And their prevalence is increasing at alarming rates in almost every developed country. Caloric sweeteners are a key contributor to this trend, as they are included in various packaged food and beverage products to make them more palatable to consumers. In many cases, no-calorie or low-calorie substitutes can be used in foods and beverages in place of sucrose or fructose. Even so, these compounds impart sweetness differently from caloric sweeteners, and a number of consumers fail to view them as suitable alternatives. Moreover, such compounds may be difficult to incorporate into certain products. In some instances, they may be used as partial replacements for caloric sweeteners, but their mere presence can cause many consumers to perceive unpleasant off-tastes including, astringency, bitterness, and metallic and licorice tastes. Thus, lower-calorie sweeteners face certain challenges to their adoption.

Sweetness enhancement provides an alternative approach to overcoming some of adoption challenges faced by lower-calorie sweeteners. Such compounds can be used in combination with sucrose or fructose to enhance their sweetness, thereby permitting the use of lower quantities of such caloric sweeteners in various food or beverage products. But, in addition to enhancing the perceived sweetness of the primary sweetener, such compounds nevertheless alter the perceived taste of the sweetener. Thus, many consumers find that it is less pleasurable to consume such sweetness-enhanced products in comparison to unenhanced alternatives having higher calories. Thus, there is a continuing need to discover compounds that enhance the sweetness of caloric sweeteners without altering their perceived taste in a way that detracts from the pleasure that consumers experience in eating or drinking products containing such sweeteners.

### SUMMARY

The present disclosure relates to the discovery that lactase enzymes, used in combination with certain sweetness enhancers, can improve the sweetness of dairy products without requiring the addition of substantial amounts of caloric sweetener, such as sucrose of fructose.

In a first aspect, the disclosure provides a flavor-modifying compound, which is a di-alkali metal salt or a tri-alkali metal salt of a compound of formula (I): 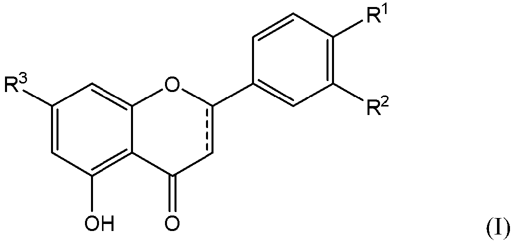 wherein R¹, R², and R³ are independently a hydrogen atom, -OH, or -OCH₃, and at least one of R¹, R², and R³ is -OH; wherein the alkali metal of the di-alkali metal salt and tri-alkali metal salt is sodium, potassium, or a combination thereof; and where the dotted line represents an optional carbon-carbon double bond.

In a second aspect, the disclosure provides a flavor-modifying compound, which is a di-alkali metal salt or a tri-alkali metal salt of a compound of formula (II): 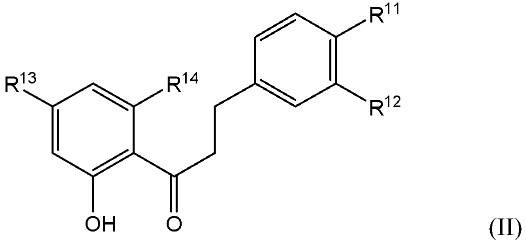 wherein R¹¹, R¹², R¹³, and R¹⁴ are independently a hydrogen atom, -OH, or -OCH₃, and at least one of R¹¹, R¹², R¹³, and R¹⁴ is -OH; and wherein the alkali metal of the di-alkali metal salt and tri-alkali metal salt is sodium, potassium, or a combination thereof.

In a third aspect, the disclosure provides a flavor-modifying compound, which is a di-alkali metal salt or a tri-alkali metal salt of a compound of formula (III): 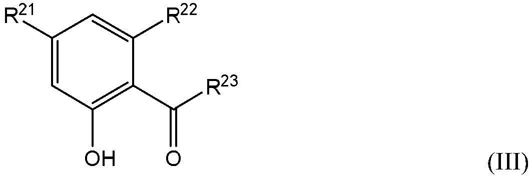 wherein R²¹ and R²² are independently a hydrogen atom, -OH, or -OCH₃, and at least one of R²¹ and R²² is -OH; R²³ is C₁₋₆ alkyl or -CH₂-R²⁴; and R²⁴ is phenyl optionally substituted from 1 to three times by -OH, -OCH₃, or a combination thereof; and wherein the alkali metal of the di-alkali metal salt and tri-alkali metal salt is sodium, potassium, or a combination thereof.

In a fourth aspect, the disclosure provides uses of any flavor-modifying compounds of the first, second, or third aspects to enhance a sweet taste of an ingestible composition. In some embodiments thereof, the ingestible composition comprises a caloric sweetener. In some other embodiments thereof, the ingestible composition comprises a non-caloric sweetener. In some embodiments, the sweetener is a high-intensity sweetener. In certain related aspects, the disclosure provides methods of enhancing a sweet taste of an ingestible composition, the methods comprising introducing any flavor-modifying compounds of the first, second, or third aspects to an ingestible composition.

In a fifth aspect, the disclosure provides uses of any flavor-modifying compounds of the first, second, or third aspects to reduce a bitter taste of an ingestible composition. In some other embodiments thereof, the ingestible composition comprises a non-caloric sweetener, such as a non-caloric sweetener having a bitter or unpleasant aftertaste. In some embodiments, the ingestible composition comprises a bitter compound, such as nomelin, limonin, caffeine, plant proteins, and the like. In some embodiments, the sweetener is a high-intensity sweetener. In certain related aspects, the disclosure provides methods of reducing a bitter taste of an ingestible composition, the methods comprising introducing any flavor-modifying compounds of the first, second, or third aspects to an ingestible composition.

In a sixth aspect, the disclosure provides uses of any flavor-modifying compounds of the first, second, or third aspects to reduce a caloric content of an ingestible composition. In some embodiments thereof, the ingestible composition comprises a caloric sweetener, but at a concentration less than that of an isosweet composition not comprising any flavor-modifying compounds disclosed herein. In certain related aspects, the disclosure provides methods of reducing a caloric content of an ingestible composition, the methods comprising introducing any flavor-modifying compounds of the first, second, or third aspects to an ingestible composition.

In a seventh aspect, the disclosure provides ingestible compositions comprising any flavor-modifying compounds of the first, second, or third aspects. In some embodiments, the ingestible composition is not a naturally occurring composition. In some embodiments, the ingestible composition comprises a sweetener. In some embodiments, the sweetener is a caloric sweetener, such as sucrose, fructose, glucose, xylitol, erythritol, or combinations thereof. In some embodiments, the sweetener is a non-caloric sweetener, such as a steviol glycoside, a mogroside, aspartame, sucralose, acesulfame K, saccharin, or any combinations thereof. In some embodiments, the ingestible composition comprises one or more high-intensity sweeteners.

In an eighth aspect, the disclosure provides flavored products comprising any ingestible compositions of the seventh aspect. In some embodiment, the flavored products are beverage products, such as soda, flavored water, tea, and the like. In some other embodiments, the flavored products are food products, such as yogurt.

Further aspects, and embodiments thereof, are set forth below in the Detailed Description, the Abstract, and the Claims.

### DETAILED DESCRIPTION

The following Detailed Description sets forth various aspects and embodiments provided herein. The description is to be read from the perspective of the person of ordinary skill in the relevant art. Therefore, information that is well known to such ordinarily skilled artisans is not necessarily included.

### Definitions

The following terms and phrases have the meanings indicated below, unless otherwise provided herein. This disclosure may employ other terms and phrases not expressly defined herein. Such other terms and phrases have the meanings that they would possess within the context of this disclosure to those of ordinary skill in the art. In some instances, a term or phrase may be defined in the singular or plural. In such instances, it is understood that any term in the singular may include its plural counterpart and vice versa, unless expressly indicated to the contrary

As used herein, "sweetener" refers to a compound or a comestibly acceptable salt thereof that elicits a detectable sweet flavor in a subject, such as a compound that activates a human T1R2 or T1R3 receptor in the course of in vitro screening or that is reported to be sweet via sensory testing by human subjects.

As used herein, "Cₐ to C_{b}" or "C_{a-b}" in which "a" and "b" are integers, refer to the number of carbon atoms in the specified group. That is, the group can contain from "a" to "b", inclusive, carbon atoms. Thus, for example, a "C₁ to C₄ alkyl" or "C₁₋₄ alkyl" group refers to all alkyl groups having from 1 to 4 carbons, that is, -CH₃, -CH₂CH₃, -CH₂CH₂CH₃, -CH(CH₃)₂, -CH₂CH₂CH₂CH₃, -CH(CH₃)CH₂CH₃, and -C(CH₃)₃.

As used herein, "alkyl" means a straight or branched hydrocarbon chain that is fully saturated (i.e., contains no double or triple bonds). In some embodiments, an alkyl group has 1 to 20 carbon atoms (whenever it appears herein, a numerical range such as "1 to 20" refers to each integer in the given range; *e.g.*, "1 to 20 carbon atoms" means that the alkyl group may consist of 1 carbon atom, 2 carbon atoms, 3 carbon atoms, *etc.*, up to and including 20 carbon atoms, although the present definition also covers the occurrence of the term "alkyl" where no numerical range is designated). The alkyl group may also be a medium size alkyl having 1 to 9 carbon atoms. The alkyl group could also be a lower alkyl having 1 to 4 carbon atoms. The alkyl group may be designated as "C₁₋₄ alkyl" or similar designations. By way of example only, "C₁₋₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl, and the like. Unless indicated to the contrary, the term "alkyl" refers to a group that is not further substituted.

It is to be understood that certain radical naming conventions can include either a mono-radical or a di-radical, depending on the context. For example, where a substituent requires two points of attachment to the rest of the molecule, it is understood that the substituent is a di-radical. For example, a substituent identified as alkyl that requires two points of attachment includes di-radicals such as -CH₂-, -CH₂CH₂-, -CH₂CH(CH₃)CH₂-, and the like.

Wherever a substituent is depicted as a di-radical (i.e., has two points of attachment to the rest of the molecule), it is to be understood that the substituent can be attached in any directional configuration unless otherwise indicated. Thus, for example, a substituent depicted as -AE- or 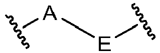 includes the substituent being oriented such that the A is attached at the leftmost attachment point of the molecule as well as the case in which A is attached at the rightmost attachment point of the molecule.

As used herein, the singular forms "a," "an," and "the" include plural referents unless the context clearly dictates otherwise. For example, reference to "a substituent" encompasses a single substituent as well as two or more substituents, and the like.

As used herein, "for example," "for instance," "such as," or "including" are meant to introduce examples that further clarify more general subject matter. Unless otherwise expressly indicated, such examples are provided only as an aid for understanding embodiments illustrated in the present disclosure, and are not meant to be limiting in any fashion. Nor do these phrases indicate any kind of preference for the disclosed embodiment.

As used herein, "comprise" or "comprises" or "comprising" or "comprised of" refer to groups that are open, meaning that the group can include additional members in addition to those expressly recited. For example, the phrase, "comprises A" means that A must be present, but that other members can be present too. The terms "include," "have," and "composed of" and their grammatical variants have the same meaning. In contrast, "consist of" or "consists of" or "consisting of" refer to groups that are closed. For example, the phrase "consists of A" means that A and only A is present.

As used herein, "optionally" means that the subsequently described event(s) may or may not occur. In some embodiments, the optional event does not occur. In some other embodiments, the optional event does occur one or more times.

As used herein, "or" is to be given its broadest reasonable interpretation, and is not to be limited to an either/or construction. Thus, the phrase "comprising A or B" means that A can be present and not B, or that B is present and not A, or that A and B are both present. Further, if A, for example, defines a class that can have multiple members, e.g., A₁ and A₂, then one or more members of the class can be present concurrently.

Points of attachment for groups are generally indicated by a terminal dash (-) or by an asterisk (*). For example, a group such as *-CH₂-CH₃ or -CH₂-CH₃ both represent an ethyl group.

Chemical structures are often shown using the "skeletal" format, such that carbon atoms are not explicitly shown, and hydrogen atoms attached to carbon atoms are omitted entirely. For example, the structure 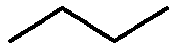 represents butane (i.e., n-butane). Furthermore, aromatic groups, such as benzene, are represented by showing one of the contributing resonance structures. For example, the structure 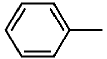 represents toluene.

Other terms are defined in other portions of this description, even though not included in this subsection.

### Flavor-Modifying Compounds

In certain aspects, the disclosure provides a flavor-modifying compound, which is a di-alkali metal salt or a tri-alkali metal salt of a compound of formula (I): 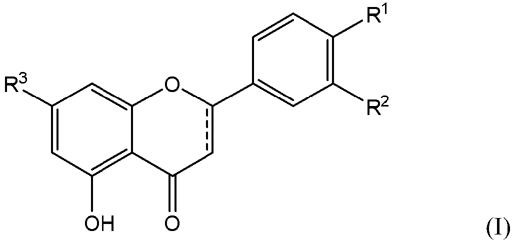 wherein R¹, R², and R³ are independently a hydrogen atom, -OH, or -OCH₃, and at least one of R¹, R², and R³ is -OH; wherein the alkali metal of the di-alkali metal salt and tri-alkali metal salt is sodium, potassium, or a combination thereof; and where the dotted line represents an optional carbon-carbon double bond.

R¹, R², and R³ can have any suitable value, according to the options set forth above. In some embodiments, R³ is -OH. In some embodiments, one of R¹ and R² is -OH, and the other is a hydrogen atom, -OH, or -OCH₃.

In some instances, the values of R¹ and R² can depend on whether the optional carbon-carbon double bond is present or absent. In some embodiments, when the optional carbon-carbon double bond is absent R¹ is a hydrogen atom or -OCH₃. In some further such embodiments, when the optional carbon-carbon double bond is absent R¹ is a hydrogen atom.
In some alternate embodiments, when the optional carbon-carbon double bond is absent R¹ is -OCH₃.

The optional carbon-carbon double bond can be present or absent. In some embodiments, it is present and the ring contains the optional carbon-carbon double bond. In other embodiments, it is absent and the ring does not contain the optional carbon-carbon double bond.

In some embodiments, the compound of formula (I) is naringenin, where R¹ and R³ are -OH, R² is a hydrogen atom, and the optional carbon-carbon double bond is absent.

The flavor-modifying compounds are di-alkali metal salts or tri-alkali metal salts. The term "salt" refers both to: (a) solid state forms, where the dianion or trianion of the compound of formula (I) is disposed in a solid-state matrix with sodium cations, potassium cations, or a combination thereof (which, in some embodiments, such solids are suspended in a liquid matrix, such as an aqueous matrix), and (b) solvated forms, where the dianion or trianion of the compound of formula (I) is disposed in a liquid medium (such as an aqueous medium) where there are a sufficient number is sodium cations, potassium cations, or a combination thereof to create neutral charge balance. Note that the molar ratio of cation to anion may not be exact. For example, a "di-alkali metal salt" means that the effective charge of the anion is about -2, and that there are about 2 moles of sodium or potassium cations present to balance the negative charge of the anion. In like manner, a "tri-alkali metal salt" means that the effective charge of the anion is about -3, and that there are about 3 moles of sodium or potassium cations present to balance the negative charge of the anion.

The flavor-modifying compounds can be any suitable di-alkali metal salt or tri-alkali metal salt of sodium, potassium, or a combination thereof. In some embodiments, the flavor-modifying compound is a di-sodium salt. In some embodiments, the flavor-modifying compound is a di-sodium salt. In some embodiments, the flavor-modifying compound is a tri-sodium salt. In some embodiments, the flavor-modifying compound is a di-potassium salt. In some embodiments, the flavor-modifying compound is a tri-potassium salt.

In certain aspects, the disclosure provides a flavor-modifying compound, which is a di-alkali metal salt or a tri-alkali metal salt of a compound of formula (II): 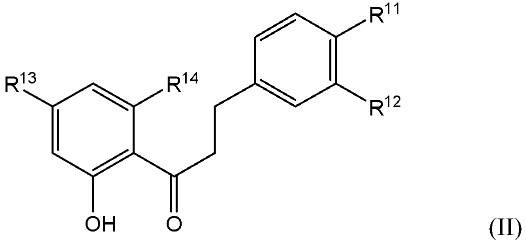 wherein R¹¹, R¹², R¹³, and R¹⁴ are independently a hydrogen atom, -OH, or -OCH₃, and at least one of R¹¹, R¹², R¹³, and R¹⁴ is -OH; and wherein the alkali metal of the di-alkali metal salt and tri-alkali metal salt is sodium, potassium, or a combination thereof.

R¹¹ and R¹² can have any suitable values within the parameters set forth above. In some embodiments, both R¹¹ and R¹² are a hydrogen atom. In some other embodiments, R¹¹ is -OH. In some such embodiments, R¹¹ is -OH and R¹² is a hydrogen atom. In some embodiments, R¹¹ is -OCH₃. In some such embodiments, R¹¹ is -OCH₃ and R¹² is a hydrogen atom or -OH.

R¹³ can have any suitable value within the parameters set forth above. In some embodiments, R¹³ is a hydrogen atom. In some embodiments, R¹³ is -OH. In some embodiments, R¹³ is -OCH₃.

R¹⁴ can have any suitable value within the parameters set forth above. In some embodiments, R¹⁴ is a hydrogen atom. In some embodiments, R¹⁴ is -OH. In some embodiments, R¹⁴ is -OCH₃.

In some embodiments, the compound of formula (II) is phloretin.

The flavor-modifying compounds are di-alkali metal salts or tri-alkali metal salts. The term "salt" refers both to: (a) solid state forms, where the dianion or trianion of the compound of formula (I) is disposed in a solid-state matrix with sodium cations, potassium cations, or a combination thereof (which, in some embodiments, such solids are suspended in a liquid matrix, such as an aqueous matrix), and (b) solvated forms, where the dianion or trianion of the compound of formula (I) is disposed in a liquid medium (such as an aqueous medium) where there are a sufficient number is sodium cations, potassium cations, or a combination thereof to create neutral charge balance. Note that the molar ratio of cation to anion may not be exact. For example, a "di-alkali metal salt" means that the effective charge of the anion is about -2, and that there are about 2 moles of sodium or potassium cations present to balance the negative charge of the anion. In like manner, a "tri-alkali metal salt" means that the effective charge of the anion is about -3, and that there are about 3 moles of sodium or potassium cations present to balance the negative charge of the anion.

The flavor-modifying compounds can be any suitable di-alkali metal salt or tri-alkali metal salt of sodium, potassium, or a combination thereof. In some embodiments, the flavor-modifying compound is a di-sodium salt. In some embodiments, the flavor-modifying compound is a di-sodium salt. In some embodiments, the flavor-modifying compound is a tri-sodium salt. In some embodiments, the flavor-modifying compound is a di-potassium salt. In some embodiments, the flavor-modifying compound is a tri-potassium salt.

In certain aspects, the disclosure provides a flavor-modifying compound, which is a di-alkali metal salt or a tri-alkali metal salt of a compound of formula (III): 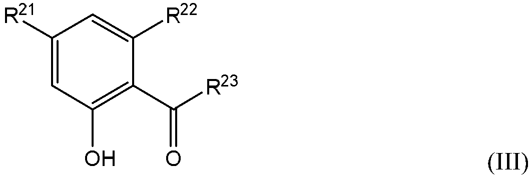 wherein R²¹ and R²² are independently a hydrogen atom, -OH, or -OCH₃, and at least one of R²¹ and R²² is -OH; R²³ is C₁₋₆ alkyl or -CH₂-R²⁴; and R²⁴ is phenyl optionally substituted from 1 to three times by -OH, -OCH₃, or a combination thereof; and wherein the alkali metal of the di-alkali metal salt and tri-alkali metal salt is sodium, potassium, or a combination thereof.

R²¹ and R²² can have any suitable values within the parameters set forth above, although at least one is -OH. In some embodiments, R²¹ is a hydrogen atom. In some embodiments, R²¹ is -OH. In some such embodiments, R²¹ is -CH₃. In some embodiments, R²² is a hydrogen atom. In some embodiments, R²² is -OH. In some such embodiments, R²² is -CH₃.

R²³ can have any suitable value within the parameters set forth above. In some embodiments, R²³ is C₁₋₆ alkyl, such as methyl, ethyl, propyl, butyl, pentyl, 2-methylpropyl, or 3-methylbutyl. In some embodiments, R²³ is -CH₂-R²⁴; and R²⁴ is phenyl optionally substituted from 1 to three times by -OH, -OCH₃, or a combination thereof. R²⁴ can have any suitable value, such as an unsubstituted phenyl group or a phenyl group substituted at the 3-position, the 4-position, or both the 3-position and the 4-position by -OH, -OCH₃, or a combination thereof.

Table 1 sets forth non-limiting examples of compounds of formula (I), compounds of formula (II), and compounds of formula (III), which can form di- or tri-alkali metal salts.

**Table 1**

| **Cmpd** | **Structure** | **Name** |
|---|---|---|
| 1 | 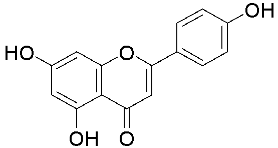 | Apigenin |
| 2 | 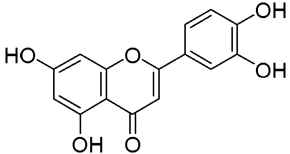 | Luteolin |
| 3 | 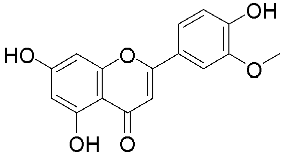 | Chrysoeriol |
| 4 | 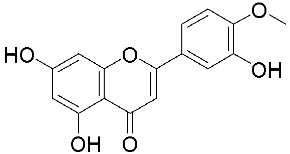 | Diosmetin |
| 5 | 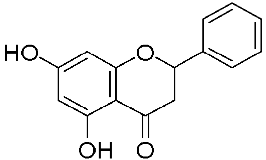 | Pinocembrin |
| 6 | 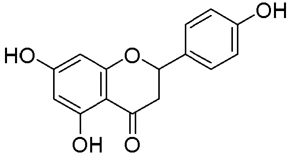 | Naringenin |
| 7 | 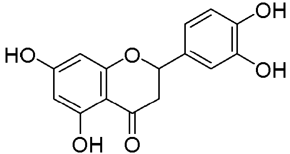 | Eriodictyol |
| 8 | 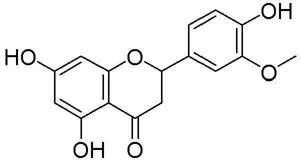 | Homoeriodictyol |
| 9 | 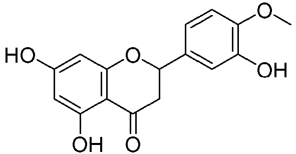 | Hesperetin |
| 10 | 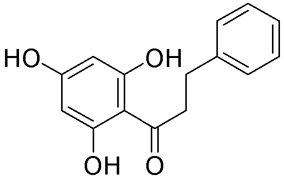 | 3-phenyl-1-(2,4,6-trihydroxyphenyl)propan-1-one |
| 11 | 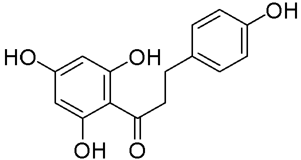 | Phloretin |
| 12 | 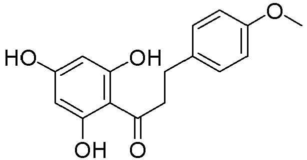 | 3-(4-methoxyphenyl)-1-(2,4,6-trihydroxyphenyl)propan-1-one |
| 13 | 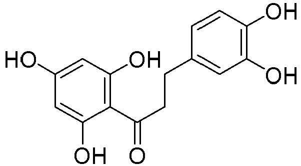 | 3-(3,4-dihydroxyphenyl)-1-(2,4,6-trihydroxyphenyl)propan-1-one |
| 14 | 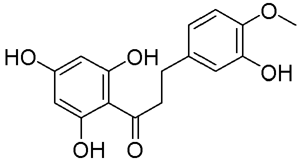 | 3-(3-hydroxy-4-methoxyphenyl)-1-(2,4,6-trihydroxyphenyl)propan-1-one |
| 15 | 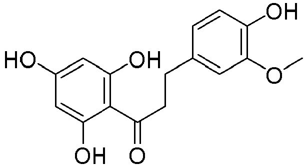 | 3-(4-hydroxy-3-methoxyphenyl)-1-(2,4,6-trihydroxyphenyl)propan-1-one |
| 16 | 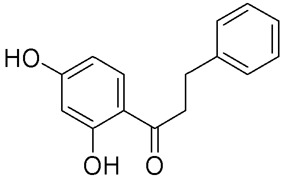 | 1-(2,4-dihydroxyphenyl)-3-phenylpropan-1-one |
| 17 | 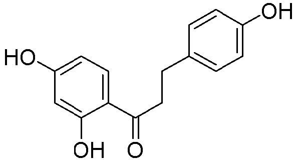 | 1-(2,4-dihydroxyphenyl)-3-(4-hydroxyphenyl)propan-1-one |
| 18 | 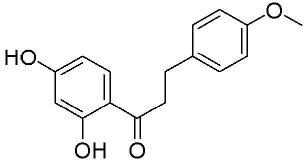 | 1-(2,4-dihydroxyphenyl)-3-(4-methoxyphenyl)propan-1-one |
| 19 | 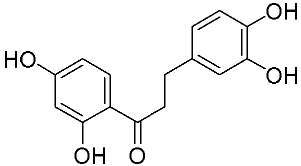 | 1-(2,4-dihydroxyphenyl)-3-(3,4-dihydroxyphenyl)propan-1-one |
| 20 | 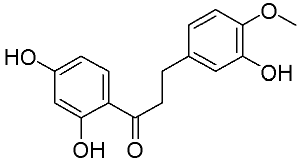 | 1-(2,4-dihydroxyphenyl)-3-(3-hydroxy-4-methoxyphenyl)propan-1-one |
| 21 | 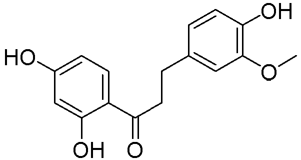 | 1-(2,4-dihydroxyphenyl)-3-(4-hydroxy-3-methoxyphenyl)propan-1-one |
| 22 | 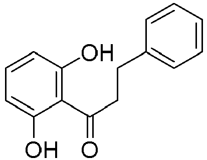 | 1-(2,6-dihydroxyphenyl)-3-phenylpropan-1-one |
| 23 | 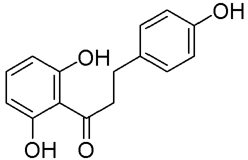 | 1-(2,6-dihydroxyphenyl)-3-(4-hydroxyphenyl)propan-1-one |
| 24 | 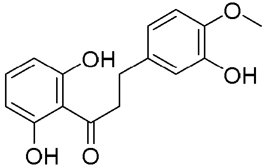 | 1-(2,6-dihydroxyphenyl)-3-(3-hydroxy-4-methoxyphenyl)propan-1-one |
| 25 | 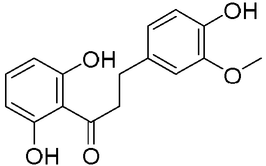 | 1-(2,6-dihydroxyphenyl)-3-(4-hydroxy-3-methoxyphenyl)propan-1-one |
| 26 | 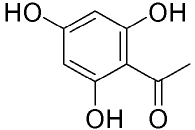 | 1-(2,4,6-trihydroxyphenyl)ethan-1-one |
| 27 | 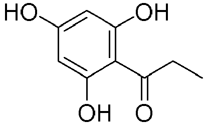 | 1-(2,4,6-trihydroxyphenyl)propan-1-one |
| 28 | 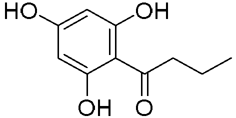 | 1-(2,4,6-trihydroxyphenyl)butan-1-one |
| 29 | 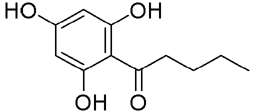 | 1-(2,4,6-trihydroxyphenyl)pentan-1-one |
| 30 | 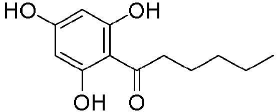 | 1-(2,4,6-trihydroxyphenyl)hexan-1-one |
| 31 | 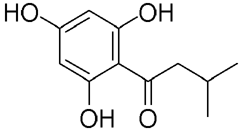 | 3-methyl-1-(2,4,6-trihydroxyphenyl)butan-1-one |
| 32 | 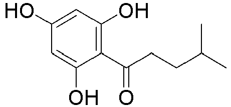 | 4-methyl-1-(2,4,6-trihydroxyphenyl)pentan-1-one |
| 33 | 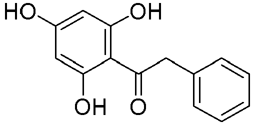 | 2-phenyl-1-(2,4,6-trihydroxyphenyl)ethan-1-one |
| 34 | 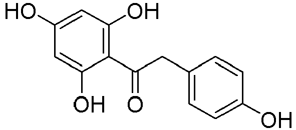 | 2-(4-hydroxyphenyl)-1-(2,4,6-trihydroxyphenyl)ethan-1-one |
| 35 | 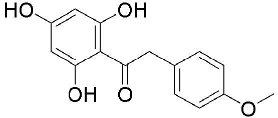 | 2-(4-methoxyphenyl)-1-(2,4,6-trihydroxyphenyl)ethan-1-one |
| 36 | 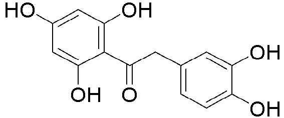 | 2-(3,4-dihydroxyphenyl)-1-(2,4,6-trihydroxyphenyl)ethan-1-one |
| 37 | 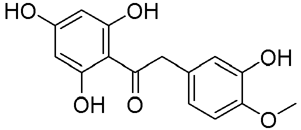 | 2-(3-hydroxy-4-methoxyphenyl)-1-(2,4,6-trihydroxyphenyl)ethan-1-one |
| 38 | 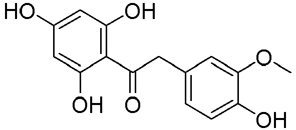 | 2-(4-hydroxy-3-methoxyphenyl)-1-(2,4,6-trihydroxyphenyl)ethan-1-one |
| 39 | 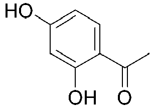 | 1-(2,4-dihydroxyphenyl)ethan-1-one |
| 40 | 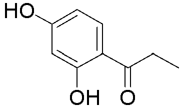 | 1-(2,4-dihydroxyphenyl)propan-1-one |
| 41 | 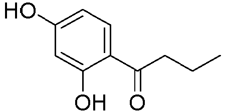 | 1-(2,4-dihydroxyphenyl)butan-1-one |
| 42 | 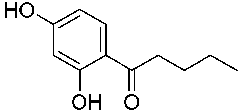 | 1-(2,4-dihydroxyphenyl)pentan-1-one |
| 43 | 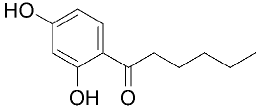 | 1-(2,4-dihydroxyphenyl)hexan-1-one |
| 44 | 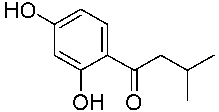 | 1-(2,4-dihydroxyphenyl)-3-methylbutan-1-one |
| 45 | 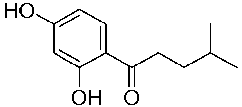 | 1-(2,4-dihydroxyphenyl)-4-methylpentan-1-one |
| 46 | 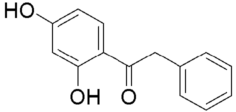 | 1-(2,4-dihydroxyphenyl)-2-phenylethan-1-one |
| 47 | 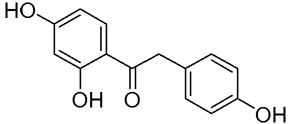 | 1-(2,4-dihydroxyphenyl)-2-(4-hydroxyphenyl)ethan-1-one |
| 48 | 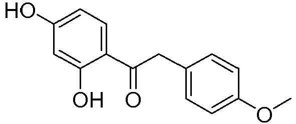 | 1-(2,4-dihydroxyphenyl)-2-(4-hydroxyphenyl)ethan-1-one |
| 49 | 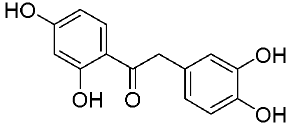 | 1-(2,4-dihydroxyphenyl)-2-(3,4-dihydroxyphenyl)ethan-1-one |
| 50 | 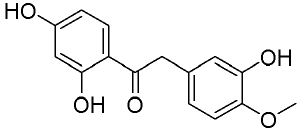 | 1-(2,4-dihydroxyphenyl)-2-(3-hydroxy-4-methoxyphenyl)ethan-1-one |
| 51 | 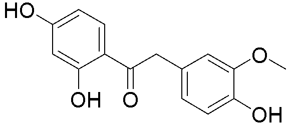 | 1-(2,4-dihydroxyphenyl)-2-(4-hydroxy-3-methoxyphenyl)ethan-1-one |

Note that some compounds in Table 1 exhibit stereochemistry. In instances where the stereochemistry is not explicitly shown in the structure, the structure includes the pure (R) form, the pure (S) form, and any racemic mixtures of the two forms.

Isotopes may be present in the compounds described. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise.

### Uses and Methods

In certain aspects, the disclosure provides uses of any flavor-modifying compounds of the preceding aspects to enhance a sweet taste of an ingestible composition. In some embodiments thereof, the ingestible composition comprises a caloric sweetener. In some other embodiments thereof, the ingestible composition comprises a non-caloric sweetener. In some embodiments, the sweetener is a high-intensity sweetener. In certain related aspects, the disclosure provides methods of enhancing a sweet taste of an ingestible composition, the methods comprising introducing any flavor-modifying compounds of the preceding aspects to an ingestible composition. In some embodiments, the flavor-modifying compounds are used or introduced to the ingestible composition at a concentration ranging from 1 ppm to 1000 ppm, or from 5 ppm to 1000 ppm, or from 10 ppm to 1000 ppm, or from 20 ppm to 1000 ppm, or from 50 ppm to 1000 ppm, or from 100 ppm to 1000 ppm, or from 200 ppm to 800 ppm, based on the total weight of the ingestible composition. In some embodiments, the flavor-modifying compound is a disodium salt of naringenin. In some embodiments, the flavor-modifying compound is a dipotassium salt of naringenin. In some embodiments, the flavor-modifying compound is a disodium salt of phloretin. In some embodiments, the flavor-modifying compound is a dipotassium salt of phloretin.

In certain aspects, the disclosure provides uses of any flavor-modifying compounds of the preceding aspects to reduce a bitter taste of an ingestible composition. In some other embodiments thereof, the ingestible composition comprises a non-caloric sweetener, such as a non-caloric sweetener having a bitter or unpleasant aftertaste. In some embodiments, the ingestible composition comprises a bitter compound, such as nomelin, limonin, caffeine, non-animal proteins, pharmaceutical APIs, and the like. In some embodiments, the sweetener is a high-intensity sweetener. In certain related aspects, the disclosure provides methods of reducing a bitter taste of an ingestible composition, the methods comprising introducing any flavor-modifying compounds of the preceding aspects to an ingestible composition. In some embodiments, the flavor-modifying compounds are used or introduced to the ingestible composition at a concentration ranging from 1 ppm to 1000 ppm, or from 5 ppm to 1000 ppm, or from 10 ppm to 1000 ppm, or from 20 ppm to 1000 ppm, or from 50 ppm to 1000 ppm, or from 100 ppm to 1000 ppm, or from 200 ppm to 800 ppm, based on the total weight of the ingestible composition. In some embodiments, the flavor-modifying compound is a disodium salt of naringenin. In some embodiments, the flavor-modifying compound is a dipotassium salt of naringenin. In some embodiments, the flavor-modifying compound is a disodium salt of phloretin. In some embodiments, the flavor-modifying compound is a dipotassium salt of phloretin.

In certain aspects, the disclosure provides uses of any flavor-modifying compounds of the preceding aspects to reduce a caloric content of an ingestible composition. In some embodiments thereof, the ingestible composition comprises a caloric sweetener, but at a concentration less than that of an isosweet composition not comprising any flavor-modifying compounds disclosed herein. In certain related aspects, the disclosure provides methods of reducing a caloric content of an ingestible composition, the methods comprising introducing any flavor-modifying compounds of the preceding aspects to an ingestible composition. In some embodiments, the flavor-modifying compounds are used or introduced to the ingestible composition at a concentration ranging from 1 ppm to 1000 ppm, or from 5 ppm to 1000 ppm, or from 10 ppm to 1000 ppm, or from 20 ppm to 1000 ppm, or from 50 ppm to 1000 ppm, or from 100 ppm to 1000 ppm, or from 200 ppm to 800 ppm, based on the total weight of the ingestible composition. In some embodiments, the flavor-modifying compound is a disodium salt of naringenin. In some embodiments, the flavor-modifying compound is a dipotassium salt of naringenin. In some embodiments, the flavor-modifying compound is a disodium salt of phloretin. In some embodiments, the flavor-modifying compound is a dipotassium salt of phloretin.

### Ingestible Compositions

The foregoing uses and methods generally involve the use of the flavor-modifying compounds in a composition containing one or more additional ingredients. For example, in at least one aspect, the disclosure provides compositions comprising any flavor-modifying compounds of the foregoing aspects, including any embodiments or combination of embodiments thereof, as set forth above, wherein the flavor-modifying compounds make up at least 50% by weight of the compositions on a dry weight basis (e.g., based on the total weight of the composition excluding the weight of any liquid carrier). In a related aspect, the disclosure provides solid-state compositions comprising any flavor-modifying compounds of the foregoing aspects, including any embodiments or combination of embodiments thereof, as set forth above, wherein the flavor-modifying compounds make up at least 50% by weight of the solid-state compositions, based on the total weight of composition. In another related aspect, the disclosure provides ingestible compositions comprising any flavor-modifying compounds of the foregoing aspects, including any embodiments or combination of embodiments thereof, as set forth above, wherein the ingestible compositions comprise a sweetener. In some such embodiments, the sweetener is a caloric sweetener, such as sucrose, fructose (e.g., high-fructose corn syrup), glucose, or combinations thereof.

In certain embodiments of any aspects and embodiments set forth herein that refer to an ingestible composition, the ingestible composition is a non-naturally-occurring product, such as a composition specifically manufactured for the production of a flavored product, such as a flavored food or beverage product, or a flavored pharmaceutical product.

In general, flavor-modifying compounds as disclosed and described herein, individually or in combination, can be provided in a composition, such as an ingestible composition. In one embodiment, the flavor-modifying compounds as disclosed and described herein, individually or in combination, can impart a more sugar-like temporal profile or flavor profile to a sweetener composition by combining one or more of the compounds as disclosed and described herein with one or more sweeteners in the sweetener composition. In another embodiment, the flavor-modifying compounds as disclosed and described herein, individually or in combination, can increase or enhance the sweet taste of a composition by contacting the composition thereof with the compounds as disclosed and described herein to form a modified composition.

Thus, in some embodiments, the compositions set forth in any of the foregoing aspects (including in any uses or methods), comprise a flavor-modifying compound and a sweetener. In some embodiments, the composition further comprises a vehicle. In some embodiments, the vehicle is water. In some embodiments, the flavor-modifying compound is present at a concentration at or below its sweetness recognition threshold.

In some embodiments, where the ingestible composition comprises a sweetener, the sweetener is present in an amount ranging from 0.1% to 12% by weight. In some embodiments, the sweetener is present in an amount ranging from 0.2% to 10% by weight. In some embodiments, the sweetener is present in an amount ranging from 0.3% to 8% by weight. In some embodiments, the sweetener is present in an amount ranging from 0.4% to 6% by weight. In some embodiments, the sweetener is present in an amount ranging from 0.5% to 5% by weight. In some embodiments, the sweetener is present in an amount ranging from 1% to 2% by weight. In some embodiments, the sweetener is present in an amount ranging from 0.1% to 5% by weight. In some embodiments, the sweetener is present in an amount ranging from 0.1% to 4% by weight. In some embodiments, the sweetener is present in an amount ranging from 0.1% to 3% by weight. In some embodiments, the sweetener is present in an amount ranging from 0.1% to 2% by weight. In some embodiments, the sweetener is present in an amount ranging from 0.1% to 1% by weight. In some embodiments, the sweetener is present in an amount ranging from 0.1% to 0.5% by weight. In some embodiments, the sweetener is present in an amount ranging from 0.5% to 10% by weight. In some embodiments, the sweetener is present in an amount ranging from 2% to 8% by weight. In some further embodiments of the embodiments set forth in this paragraph, the sweetener is sucrose, fructose, glucose, xylitol, erythritol, allulose, or combinations thereof.

In some other embodiments, the sweetener is present in an amount ranging from 10 ppm to 1000 ppm. In some embodiments, the sweetener is present in an amount ranging from 20 ppm to 800 ppm. In some embodiments, the sweetener is present in an amount ranging from 30 ppm to 600 ppm. In some embodiments, the sweetener is present in an amount ranging from 40 ppm to 500 ppm. In some embodiments, the sweetener is present in an amount ranging from 50 ppm to 400 ppm. In some embodiments, the sweetener is present in an amount ranging from 50 ppm to 300 ppm. In some embodiments, the sweetener is present in an amount ranging from 50 ppm to 200 ppm. In some embodiments, the sweetener is present in an amount ranging from 50 ppm to 150 ppm. In some further embodiments of the embodiments set forth in this paragraph, the sweetener is a steviol glycoside, such as renaudioside A, rebaudioside D, rebaudioside M, rebaudioside E, or any combinations thereof. In some embodiments, the sweetener is a mogroside, such as mogroside III, mogroside IV, mogroside V, siamenoside I, isomogroside V, mogroside IV_{E}, isomogroside IV, mogroside III_{E}, 11-oxomogroside V, the 1,6-α isomer of siamenoside I, and any combinations thereof. In some embodiments, the sweetener is sucralose.

In addition to what is set forth above, the compositions can include any suitable sweeteners or combination of sweeteners. In some embodiments, the sweetener is a common saccharide sweeteners, such as sucrose, fructose, glucose, and sweetener compositions comprising natural sugars, such as corn syrup (including high fructose corn syrup) or other syrups or sweetener concentrates derived from natural fruit and vegetable sources. In some embodiments, the sweetener is sucrose, fructose, or a combination thereof. In some embodiments, the sweetener is sucrose. In some other embodiments, the sweetener is selected from rare natural sugars including D-allose, D-psicose, L-ribose, D-tagatose, L-glucose, L-fucose, L-arbinose, D-turanose, and D-leucrose. In some embodiments, the sweetener is selected from semi-synthetic "sugar alcohol" sweeteners such as erythritol, isomalt, lactitol, mannitol, sorbitol, xylitol, maltodextrin, and the like. In some embodiments, the sweetener is selected from artificial sweeteners such as aspartame, saccharin, acesulfame-K, cyclamate, sucralose, and alitame. In some embodiments, the sweetener is selected from the group consisting of cyclamic acid, mogroside, tagatose, maltose, galactose, mannose, sucrose, fructose, lactose, neotame and other aspartame derivatives, glucose, D-tryptophan, glycine, maltitol, lactitol, isomalt, hydrogenated glucose syrup (HGS), hydrogenated starch hydrolyzate (HSH), stevioside, rebaudioside A, other sweet Stevia-based glycosides, chemically modified steviol glycosides (such as glucosylated steviol glycosides), mogrosides, chemically modified mogrosides (such as glucosylated mogrosides), carrelame and other guanidine-based sweeteners. In some embodiments, the sweetener is a combination of two or more of the sweeteners set forth in this paragraph. In some embodiments, the sweetener may combinations of two, three, four or five sweeteners as disclosed herein. In some embodiments, the sweetener may be a sugar. In some embodiments, the sweetener may be a combination of one or more sugars and other natural and artificial sweeteners. In some embodiments, the sweetener is a sugar. In some embodiments, the sugar is cane sugar. In some embodiments, the sugar is beet sugar. In some embodiments, the sugar may be sucrose, fructose, glucose or combinations thereof. In some embodiments, the sugar may be sucrose. In some embodiments, the sugar may be a combination of fructose and glucose.

The sweetener can also include, for example, sweetener compositions comprising one or more natural or synthetic carbohydrate, such as corn syrup, high fructose corn syrup, high maltose corn syrup, glucose syrup, sucralose syrup, hydrogenated glucose syrup (HGS), hydrogenated starch hydrolyzate (HSH), or other syrups or sweetener concentrates derived from natural fruit and vegetable sources, or semi-synthetic "sugar alcohol" sweeteners such as polyols. Non-limiting examples of polyols in some embodiments include erythritol, maltitol, mannitol, sorbitol, lactitol, xylitol, isomalt, propylene glycol, glycerol (glycerin), threitol, galactitol, palatinose, reduced isomalto-oligosaccharides, reduced xylooligosaccharides, reduced gentio-oligosaccharides, reduced maltose syrup, reduced glucose syrup, isomaltulose, maltodextrin, and the like, and sugar alcohols or any other carbohydrates or combinations thereof capable of being reduced which do not adversely affect taste.

The sweetener may be a natural or synthetic sweetener that includes, but is not limited to, agave inulin, agave nectar, agave syrup, amazake, brazzein, brown rice syrup, coconut crystals, coconut sugars, coconut syrup, date sugar, fructans (also referred to as inulin fiber, fructo-oligosaccharides, or oligo-fructose), green stevia powder, stevia rebaudiana, rebaudioside A, rebaudioside B, rebaudioside C, rebaudioside D, rebaudioside E, rebaudioside F, rebaudioside I, rebaudioside H, rebaudioside L, rebaudioside K, rebaudioside J, rebaudioside N, rebaudioside O, rebaudioside M and other sweet stevia-based glycosides, stevioside, stevioside extracts, honey, Jerusalem artichoke syrup, licorice root, luo han guo (fruit, powder, or extracts), lucuma (fruit, powder, or extracts), maple sap (including, for example, sap extracted from *Acer saccharum, Acer nigrum, Acer rubrum, Acer saccharinum, Acer platanoides, Acer negundo, Acer macrophyllum, Acer grandidentatum, Acer glabrum, Acer mono*)*,* maple syrup, maple sugar, walnut sap (including, for example, sap extracted from *Juglans cinerea, Juglans nigra, Juglans ailatifolia, Juglans regia*)*,* birch sap (including, for example, sap extracted from *Betula papyrifera, Betula alleghaniensis, Betula lenta, Betula nigra, Betula populifolia, Betula pendula*)*,* sycamore sap (such as, for example, sap extracted from *Platanus occidentalis*)*,* ironwood sap (such as, for example, sap extracted from *Ostrya virginiana*)*,* mascobado, molasses (such as, for example, blackstrap molasses), molasses sugar, monatin, monellin, cane sugar (also referred to as natural sugar, unrefined cane sugar, or sucrose), palm sugar, panocha, piloncillo, rapadura, raw sugar, rice syrup, sorghum, sorghum syrup, cassava syrup (also referred to as tapioca syrup), thaumatin, yacon root, malt syrup, barley malt syrup, barley malt powder, beet sugar, cane sugar, crystalline juice crystals, caramel, carbitol, carob syrup, castor sugar, hydrogenated starch hydrolates, hydrolyzed can juice, hydrolyzed starch, invert sugar, anethole, arabinogalactan, arrope, syrup, P-4000, acesulfame potassium (also referred to as acesulfame K or ace-K), alitame (also referred to as aclame), advantame, aspartame, baiyunoside, neotame, benzamide derivatives, bernadame, canderel, carrelame and other guanidine-based sweeteners, vegetable fiber, corn sugar, coupling sugars, curculin, cyclamates, cyclocarioside I, demerara, dextran, dextrin, diastatic malt, dulcin, sucrol, valzin, dulcoside A, dulcoside B, emulin, enoxolone, maltodextrin, saccharin, estragole, ethyl maltol, glucin, gluconic acid, glucono-lactone, glucosamine, glucoronic acid, glycerol, glycine, glycyphillin, glycyrrhizin, glycyrrhetic acid monoglucuronide, golden sugar, yellow sugar, golden syrup, granulated sugar, gynostemma, hernandulcin, isomerized liquid sugars, jallab, chicory root dietary fiber, kynurenine derivatives (including N'-formyl-kynurenine, N'-acetyl-kynurenine, 6-chloro-kynurenine), galactitol, litesse, ligicane, lycasin, lugduname, guanidine, falernum, mabinlin I, mabinlin II, maltol, maltisorb, maltodextrin, maltotriol, mannosamine, miraculin, mizuame, mogrosides (including, for example, mogroside IV, mogroside V, and neomogroside), mukurozioside, nano sugar, naringin dihydrochalcone, neohesperidine dihydrochalcone, nib sugar, nigero-oligosaccharide, norbu, orgeat syrup, osladin, pekmez, pentadin, periandrin I, perillaldehyde, perillartine, petphyllum, phenylalanine, phlomisoside I, phlorodizin, phyllodulcin, polyglycitol syrups, polypodoside A, pterocaryoside A, pterocaryoside B, rebiana, refiners syrup, rub syrup, rubusoside, selligueain A, siamenoside I, siraitia grosvenorii, soybean oligosaccharide, Splenda, SRI oxime V, steviol glycoside, steviolbioside, stevioside, strogins 1, 2, and 4, sucronic acid, sucrononate, sugar, suosan, phloridzin, superaspartame, tetrasaccharide, threitol, treacle, trilobatin, D-tryptophan and derivatives (6-trifluoromethyl-tryptophan, 6-chloro-D-tryptophan), vanilla sugar, volemitol, birch syrup, aspartame-acesulfame, assugrin, aromedendrin-3-O-acetate, dihydrotamarixetin-3-O-acetate, and combinations or blends of any two or more thereof.

In still other embodiments, the sweetener can be a chemically or enzymatically modified natural high potency sweetener. Modified natural high potency sweeteners include glycosylated natural high potency sweetener such as glucosyl-, galactosyl-, or fructosyl-derivatives containing 1-50 glycosidic residues. Glycosylated natural high potency sweeteners may be prepared by enzymatic transglycosylation reaction catalyzed by various enzymes possessing transglycosylating activity. In some embodiments, the modified sweetener can be substituted or unsubstituted.

Additional sweeteners also include combinations of any two or more of any of the aforementioned sweeteners. In some embodiments, the sweetener may comprise combinations of two, three, four or five sweeteners as disclosed herein. In some embodiments, the sweetener may be a sugar. In some embodiments, the sweetener may be a combination of one or more sugars and other natural and artificial sweeteners. In some embodiments, the sweetener is a caloric sweetener, such as sucrose, fructose, xylitol, erythritol, or combinations thereof. In some embodiments, the ingestible compositions are free (or, in some embodiments) substantially free of stevia-derived sweeteners, such as steviol glycosides, glucosylated steviol glycosides, or rebaudiosides. For example, in some embodiments, the ingestible compositions are either free of stevia-derived sweeteners or comprise stevia-derived sweeteners in a concentration of no more than 1000 ppm, or no more than 500 ppm, or no more than 200 ppm, or no more than 100 ppm, or no more than 50 ppm, or no more than 20 ppm, or no more than 10 ppm, or no more than 5 ppm, or no more than 3 ppm, or no more than 1 ppm.

The flavor-modifying compounds can be present in the ingestible compositions in any suitable amount. In some embodiments, the flavor-modifying compounds are present in an amount sufficient to enhance the taste (e.g., enhance the sweetness, reduce the sourness, or reduce the bitterness) of the compositions. In some embodiments, the ingestible composition comprises the flavor-modifying compound in a concentration ranging from 1 ppm to 1000 ppm, or from 5 ppm to 1000 ppm, or from 10 ppm to 1000 ppm, or from 20 ppm to 1000 ppm, or from 50 ppm to 1000 ppm, or from 100 ppm to 1000 ppm, or from 200 ppm to 800 ppm, based on the total weight of the ingestible composition. In embodiments where a sweetener, such as sucrose, glucose, or fructose, are present, the weight-to-weight ratio of sweetener to the flavor-modifying compound in the ingestible composition ranges from 10:1 to 1000:1, or from 10:1 to 500:1, or from 10:1 to 100:1.

The flavor-modifying compounds can be combined in any suitable way. For example, in some embodiments, the ingestible composition or sweetener concentrate contains a single flavor-modifying compound of the embodiments set forth above, such as a disodium salt of any one of Compounds 1-51, or a dipotassium salt of any one of Compounds 1-51, or a trisodium salt of any one of Compounds 1-51, or a tripotassium salt of any one of Compounds 1-51,. In some other embodiments, the ingestible composition or sweetener concentrate contains mixtures of two or more flavor-modifying compounds of the embodiments set forth above. For example, in some embodiments, the ingestible composition comprises a disodium salt of Compound 6 and the disodium salt of Compound 11. The two flavor-modifying compounds can have any suitable weight ratio relative to each other, such as from 1:10 to 10:1.

The ingestible compositions, in certain embodiments, also comprise any additional ingredients or combination of ingredients as are commonly used in food and beverage products, including, but not limited to:
acids, including, for example citric acid, phosphoric acid, ascorbic acid, sodium acid sulfate, lactic acid, or tartaric acid;
bitter ingredients, including, for example caffeine, quinine, green tea, catechins, polyphenols, green robusta coffee extract, green coffee extract, potassium chloride, menthol, non-animal proteins (such as proteins and protein isolates derived from plants, algae, or fungi), or pharmaceutical APIs;
coloring agents, including, for example caramel color, Red #40, Yellow #5, Yellow #6, Blue #1, Red #3, purple carrot, black carrot juice, purple sweet potato, vegetable juice, fruit juice, beta carotene, turmeric curcumin, or titanium dioxide;
preservatives, including, for example sodium benzoate, potassium benzoate, potassium sorbate, sodium metabisulfate, sorbic acid, or benzoic acid;
antioxidants including, for example ascorbic acid, calcium disodium EDTA, alpha tocopherols, mixed tocopherols, rosemary extract, grape seed extract, resveratrol, or sodium hexametaphosphate;
vitamins or functional ingredients including, for example resveratrol, Co-Q10, omega 3 fatty acids, theanine, choline chloride (citocoline), fibersol, inulin (chicory root), taurine, panax ginseng extract, guanana extract, ginger extract, L-phenylalanine, L-carnitine, L-tartrate, D-glucoronolactone, inositol, bioflavonoids, Echinacea, ginko biloba, yerba mate, flax seed oil, garcinia cambogia rind extract, white tea extract, ribose, milk thistle extract, grape seed extract, pyrodixine HCl (vitamin B6), cyanoobalamin (vitamin B12), niacinamide (vitamin B3), biotin, calcium lactate, calcium pantothenate (pantothenic acid), calcium phosphate, calcium carbonate, chromium chloride, chromium polynicotinate, cupric sulfate, folic acid, ferric pyrophosphate, iron, magnesium lactate, magnesium carbonate, magnesium sulfate, monopotassium phosphate, monosodium phosphate, phosphorus, potassium iodide, potassium phosphate, riboflavin, sodium sulfate, sodium gluconate, sodium polyphosphate, sodium bicarbonate, thiamine mononitrate, vitamin D3, vitamin A palmitate, zinc gluconate, zinc lactate, or zinc sulphate;
clouding agents, including, for example ester gun, brominated vegetable oil (BVO), or sucrose acetate isobutyrate (SAIB);
buffers, including, for example sodium citrate, potassium citrate, or salt;
flavors, including, for example propylene glycol, ethyl alcohol, glycerine, gum Arabic (gum acacia), maltodextrin, modified corn starch, dextrose, natural flavor, natural flavor with other natural flavors (natural flavor WONF), natural and artificial flavors, artificial flavor, silicon dioxide, magnesium carbonate, or tricalcium phosphate; or
starches and stabilizers, including, for example pectin, xanthan gum, carboxylmethylcellulose (CMC), polysorbate 60, polysorbate 80, medium chain triglycerides, cellulose gel, cellulose gum, sodium caseinate, modified food starch, gum Arabic (gum acacia), inulin, or carrageenan.

The ingestible compositions can have any suitable pH. In some embodiments, the flavor-modifying compounds enhance the sweetness of a sweetener under a broad range of pH, e.g., from lower pH to neutral pH. The lower and neutral pH includes, but is not limited to, a pH from about 2.5 to about 8.5; from about 3.0 to about 8.0; from about 3.5 to about 7.5; and from about 4.0 to about 7. In certain embodiments, compounds as disclosed and described herein, individually or in combination, can enhance the perceived sweetness of a fixed concentration of a sweetener in taste tests at a compound concentration of about 50 µM, 40 µM, 30 µM, 20 µM, or 10 µM at both low to neutral pH value. In certain embodiments, the enhancement factor of the compounds as disclosed and described herein, individually or in combination, at the lower pH is substantially similar to the enhancement factor of the compounds at neutral pH. Such consistent sweet enhancing property under a broad range of pH allow a broad use in a wide variety of foods and beverages of the compounds as disclosed and described herein, individually or in combination.

The ingestible compositions set forth according to any of the foregoing embodiments, also include, in certain embodiments, one or more additional flavor-modifying compounds, such as compounds that enhance sweetness (e.g., hesperetin, naringenin, glucosylated steviol glycosides, etc.), compounds that block bitterness, compounds that enhance umami, compounds that reduce sourness or licorice taste or astringency, compounds that enhance saltiness, compounds that enhance a cooling effect, compounds that enhance mouthfeel, or any combinations of the foregoing.

In some embodiments, the ingestible composition comprises a sweetness enhancer. Any suitable sweetness enhancer can be used in the flavor-modifying compositions disclosed herein, including synthetic sweetness enhancers, natural sweetness enhancers, or any combinations thereof.

Examples of suitable synthetic sweetness enhancers include, but are not limited to, *N*-(1-((4-amino-2,2-dioxo-1*H*-benzo[c][1,2,6]thiadiazin-5-yl)oxy)-2-methylpropan-2-yl)isonicotinamide, or any of its comestbly acceptable salts, or any compounds set forth in U.S. Patent Nos. 8,541,421; 8,815,956; 9,834,544; 8,592,592; 8,877,922; 9,000,054; and 9,000,051, as well as U.S. Patent Application Publication No. 2017/0119032.

Suitable examples of natural sweetness enhancers include, but are not limited to, hesperitin dihydrochalcone, hesperitin dihydrochalcone-4'-O'glucoside, neohesperitin dihydrochalcone, brazzein, hesperidin, phyllodulcin, naringenin, naringin, phloretin, glucosylated steviol glycosides, (2R,3R)-3-acetoxy-5,7,4'-trihydroxyflavanone, (2R,3R)-3-acetoxy-5,7,3'-trihydroxy-4'-methoxyflavanone, rubusosides, thaumatin, monellin, miraculin, glycyrrhizin and comestible acceptable salts thereof (such as the monoammonium salt), naringin dihydrochalcone, myricetin, nobiletin, polymethoxyflavones, mixed methoxy- and hydroxyflavones, quercetin, certain amino acids, and the like. As used herein, the term "glucosylated steviol glycoside" refers to the product of enzymatically glucosylating natural steviol glycoside compounds. The glucosylation generally occurs through a glycosidic bond, such as an α-1,2 bond, an α-1,4 bond, an α-1.6 bond, a β-1,2 bond, a β-1,4 bond, a β-1,6 bond, and so forth.

In some embodiments of any of the preceding embodiments, the comestible composition comprises 3-((4-amino-2,2-dioxo-1*H*-benzo[*c*][1,2,6]thiadiazin-5-yl)oxy)-2,2-dimethyl-*N*-propyl-propanamide, *N*-(1-((4-amino-2,2-dioxo-1*H*-benzo[c][1,2,6]-thiadiazin-5-yl)oxy)-2-methyl-propan-2-yl)isonicotinamide, or a comestibly acceptable salt thereof. In some embodiments, the flavor-modifying composition comprises *N*-(1-((4-amino-2,2-dioxo-1*H*-benzo[c][1,2,6]thiadiazin-5-yl)oxy)-2-methyl-propan-2-yl)isonicotinamide, or a comestbly acceptable salt thereof. In some embodiments, the flavor-modifying composition comprises *N*-(1-((4-amino-2,2-dioxo-1*H*-benzo[c][1,2,6]thiadiazin-5-yl)oxy)-2-methyl-propan-2-yl)isonicotinamide,

The sweetness enhancer can be present in the flavor-modifying composition in any suitable amount relative to the flavor-modifying composition, such as from 1:1 to 500:1, or from 2:1 to 500:1, or from 5:1 to 500:1, or from 10:1 to 500:1, or from 1:1 to 300:1, or from 2:1 to 300:1, or from 5:1 to 300:1, or from 10:1 to 300:1.

In some further embodiments, the ingestible compositions comprise the flavor-modifying compound disclosed herein, according to any of the embodiments or combination of embodiments set forth above, combined with one or more umami enhancing compounds. Such umami enhancing compounds include, but are not limited to, naturally derived compounds, such as (2R,4R)-1,2,4-trihydroxy-heptadec-16-ene,
(2R,4R)-1,2,4-trihydroxy-heptadec-16-yne, [8]-gingerdioldiacetate, [10]-gingerdioldiacetate, ericamide (FEMA4877), rubemamine (FEMA4310), rubescenamine (FEMA4773) or synthetic compounds, such as any compounds set forth in U.S. Patent Nos. 8,735,081; 8,124,121; and 8,968,708, such as *N*-(heptan-4-yl)benzo-[*d*][1,3]dioxole-5-carboxamide.

In some further embodiments, the ingestible compositions comprise the flavor-modifying compound disclosed herein, according to any of the embodiments or combination of embodiments set forth above, combined with one or more cooling enhancing compounds. Such cooling enhancing compounds include, but are not limited to, naturally derived compounds, such as menthol or analogs thereof, or synthetic compounds, such as any compounds set forth in U.S. Patent Nos. 9,394,287 and 10,421,727.

In some further embodiments, the ingestible compositions comprise the flavor-modifying compound disclosed herein, according to any of the embodiments or combination of embodiments set forth above, combined with one or more bitterness blocking compounds. Such bitterness blocking compounds include, but are not limited to, naturally derived compounds, such as menthol or analogs thereof, or synthetic compounds, such as any compounds set forth in U.S. Patent Nos. 8,076,491; 8,445,692; and 9,247,759. In some embodiments, the bitterness blocking compound is 3-(1-((3,5-dimethylisoxazol-4-yl)-methyl)-1*H*-pyrazol-4-yl)-1-(3-hydroxybenzyl)-imidazolidine-2,4-dione.

In some further embodiments, the ingestible compositions comprise the flavor-modifying compound disclosed herein, according to any of the embodiments or combination of embodiments set forth above, combined with one or more sour taste modulating compounds.

In some further embodiments, the ingestible compositions comprise the flavor-modifying compound disclosed herein, according to any of the embodiments or combination of embodiments set forth above, combined with one or more mouthfeel modifying compounds, such as polymethoxy flavones or mixed hydroxy- and methoxy- flavones.

In some further embodiments, the ingestible compositions comprise the flavor-modifying compound disclosed herein, according to any of the embodiments or combination of embodiments set forth above, combined with one or more flavor masking compounds. Such flavor masking compounds include, but are not limited to, cellulosic materials, materials extracted from fungus, materials extracted from plants, citric acid, carbonic acid (or carbonates), and the like.

In some further embodiments, the flavor-modifying composition comprises one or more mouthfeel modifying compounds. Such mouthfeel modifying compounds include, but are not limited to, tannins, cellulosic materials, bamboo powder, and the like.

In some further embodiments, the flavor-modifying composition comprises one or more flavor masking compounds. Such flavor masking compounds include, but are not limited to, cellulosic materials, materials extracted from fungus, materials extracted from plants, citric acid, carbonic acid (or carbonates), and the like.

In some embodiments, the flavor-modifying compounds described above are included to improve other tastants that may be present in the comestible composition itself, or that may be included within the flavored products that employ such compositions. Such tastants include sweeteners, umami tastants, kokumi tastants, bitter tastants, sour tastants, and the like.

In some embodiments, the ingestible composition comprises a yeast extract, such as a yeast lysate. Such extracts can be obtained from any suitable yeast strain, where such extracts are suitable for human consumption. Non-limiting examples of such yeasts include: yeasts of the genus *Saccharomyces*, such as *Saccharomyces cerevisiae* or *Saccharomyces pastorianus*; yeasts of the genus *Candida*, such as *Candida utilis*; yeasts of the genus *Kluyveromyces*, such as *Kluyveromyces lactis* or *Kluyveromyces marxianus*; yeasts of the genus *Pichia* such as *Pichia pastoris*; yeasts of the genus *Debaryomyces* such as *Debaryomyces hansenii*; and yeasts of the genus *Zygosaccharomyces* such as *Zygosaccharomyces mellis*. In some embodiments, the yeast is a yeast collected after brewing beer, sake, or the like. In some embodiments, the yeast is a yeast subjected to drying treatment (dried yeast) after collection.

Such extracts can be produced by any suitable means. In general, yeast extracts or lysates are made by extracting the contents of the yeast cells from the cell wall material. In many instances, the digestive enzymes in the cells (or additional enzymes added to the composition) break down the proteins and polynucleotides in the yeast to amino acids, oligopeptides (for example, from 2 to 10 peptides), nucleotides, oligonucleotides (from 2 to 10 nucleotides), and mixtures thereof. A yeast lysate can be prepared by lysing a yeast. For example, in some embodiments, the yeast after culture is crushed or lysed by an enzymatic decomposition method, a self-digestion method, an alkaline extraction method, a hot water extraction method, an acid decomposition method, an ultrasonic crushing method, crushing with a homogenizer, a freezing-thawing method, or the like (two or more thereof may be used in combination), whereby a yeast lysate is obtained. Yeast may be cultured by a conventional method. In some embodiments, the yeast after culture is heat-treated and then treated with a lytic enzyme to obtain an enzyme lysate. The conditions for the heat treatment are, for example, 80 °C to 90 °C for 5 minutes to 30 minutes. As the lytic enzyme used for the enzymatic decomposition method, various enzymes can be used as long as they can lyse the cell wall of yeast. The reaction conditions may be set so as to be optimum or suitable for the lytic enzyme(s) to be used, and specific examples thereof can include a temperature of 50 °C to 60 °C, and a pH of 7.0 to 8.0. The reaction time is also not particularly limited, and can be, for example, 3 hours to 5 hours.

Compositions comprising yeast lysate can be obtained from a variety of commercial sources. For example, in some embodiments, the yeast lysate is provides by the flavoring additive sold under the name MODUMAX (DSM Food Specialties BV, Delft, Netherlands).

### Flavored Products and Concentrates

In certain aspects, the disclosure provides flavored products comprising any ingestible compositions of the preceding aspects. In some embodiment, the flavored products are beverage products, such as soda, flavored water, tea, and the like. In some other embodiments, the flavored products are food products, such as yogurt.

In embodiments where the flavored product is a beverage, the beverage may be selected from the group consisting of enhanced sparkling beverages, colas, lemon-lime flavored sparkling beverages, orange flavored sparkling beverages, grape flavored sparkling beverages, strawberry flavored sparkling beverages, pineapple flavored sparkling beverages, ginger-ales, root beers, fruit juices, fruit-flavored juices, juice drinks, nectars, vegetable juices, vegetable-flavored juices, sports drinks, energy drinks, enhanced water drinks, enhanced water with vitamins, near water drinks, coconut waters, tea type drinks, coffees, cocoa drinks, beverages containing milk components, beverages containing cereal extracts and smoothies. In some embodiments, the beverage may be a soft drink.

In certain embodiments of any aspects and embodiments set forth herein that refer to an flavored product, the flavored product is a non-naturally-occurring product, such as a packaged food or beverage product.

Further non-limiting examples of food and beverage products or formulations include sweet coatings, frostings, or glazes for such products or any entity included in the Soup category, the Dried Processed Food category, the Beverage category, the Ready Meal category, the Canned or Preserved Food category, the Frozen Processed Food category, the Chilled Processed Food category, the Snack Food category, the Baked Goods category, the Confectionery category, the Dairy Product category, the Ice Cream category, the Meal Replacement category, the Pasta and Noodle category, and the Sauces, Dressings, Condiments category, the Baby Food category, and/or the Spreads category.

In general, the Soup category refers to canned/preserved, dehydrated, instant, chilled, UHT and frozen soup. For the purpose of this definition soup(s) means a food prepared from meat, poultry, fish, vegetables, grains, fruit and other ingredients, cooked in a liquid which may include visible pieces of some or all of these ingredients. It may be clear (as a broth) or thick (as a chowder), smooth, pureed or chunky, ready-to-serve, semi-condensed or condensed and may be served hot or cold, as a first course or as the main course of a meal or as a between meal snack (sipped like a beverage). Soup may be used as an ingredient for preparing other meal components and may range from broths (consommé) to sauces (cream or cheese-based soups).

The Dehydrated and Culinary Food Category usually means: (i) Cooking aid products such as: powders, granules, pastes, concentrated liquid products, including concentrated bouillon, bouillon and bouillon like products in pressed cubes, tablets or powder or granulated form, which are sold separately as a finished product or as an ingredient within a product, sauces and recipe mixes (regardless of technology); (ii) Meal solutions products such as: dehydrated and freeze dried soups, including dehydrated soup mixes, dehydrated instant soups, dehydrated ready-to-cook soups, dehydrated or ambient preparations of ready-made dishes, meals and single serve entrees including pasta, potato and rice dishes; and (iii) Meal embellishment products such as: condiments, marinades, salad dressings, salad toppings, dips, breading, batter mixes, shelf stable spreads, barbecue sauces, liquid recipe mixes, concentrates, sauces or sauce mixes, including recipe mixes for salad, sold as a finished product or as an ingredient within a product, whether dehydrated, liquid or frozen.

The Beverage category usually means beverages, beverage mixes and concentrates, including but not limited to, carbonated and non-carbonated beverages, alcoholic and non-alcoholic beverages, ready to drink beverages, liquid concentrate formulations for preparing beverages such as sodas, and dry powdered beverage precursor mixes. The Beverage category also includes the alcoholic drinks, the soft drinks, sports drinks, isotonic beverages, and hot drinks. The alcoholic drinks include, but are not limited to beer, cider/perry, FABs, wine, and spirits. The soft drinks include, but are not limited to carbonates, such as colas and non-cola carbonates; fruit juice, such as juice, nectars, juice drinks and fruit flavored drinks; bottled water, which includes sparkling water, spring water and purified/table water; functional drinks, which can be carbonated or still and include sport, energy or elixir drinks; concentrates, such as liquid and powder concentrates in ready to drink measure. The drinks, either hot or cold, include, but are not limited to coffee or ice coffee, such as fresh, instant, and combined coffee; tea or ice tea, such as black, green, white, oolong, and flavored tea; and other drinks including flavor-, malt- or plant-based powders, granules, blocks or tablets mixed with milk or water.

The Snack Food category generally refers to any food that can be a light informal meal including, but not limited to Sweet and savory snacks and snack bars. Examples of snack food include, but are not limited to fruit snacks, chips/crisps, extruded snacks, tortilla/corn chips, popcorn, pretzels, nuts and other sweet and savory snacks. Examples of snack bars include, but are not limited to granola/muesli bars, breakfast bars, energy bars, fruit bars and other snack bars.

The Baked Goods category generally refers to any edible product the process of preparing which involves exposure to heat or excessive sunlight. Examples of baked goods include, but are not limited to bread, buns, cookies, muffins, cereal, toaster pastries, pastries, waffles, tortillas, biscuits, pies, bagels, tarts, quiches, cake, any baked foods, and any combination thereof.

The Ice Cream category generally refers to frozen dessert containing cream and sugar and flavoring. Examples of ice cream include, but are not limited to: impulse ice cream; take-home ice cream; frozen yoghurt and artisanal ice cream; soy, oat, bean (e.g., red bean and mung bean), and rice-based ice creams.

The Confectionery category generally refers to edible product that is sweet to the taste. Examples of confectionery include, but are not limited to candies, gelatins, chocolate confectionery, sugar confectionery, gum, and the likes and any combination products.

The Meal Replacement category generally refers to any food intended to replace the normal meals, particularly for people having health or fitness concerns. Examples of meal replacement include, but are not limited to slimming products and convalescence products.

The Ready Meal category generally refers to any food that can be served as meal without extensive preparation or processing. The ready meal includes products that have had recipe "skills" added to them by the manufacturer, resulting in a high degree of readiness, completion and convenience. Examples of ready meal include, but are not limited to canned/preserved, frozen, dried, chilled ready meals; dinner mixes; frozen pizza; chilled pizza; and prepared salads.

The Pasta and Noodle category includes any pastas and/or noodles including, but not limited to canned, dried and chilled/fresh pasta; and plain, instant, chilled, frozen and snack noodles.

The Canned/Preserved Food category includes, but is not limited to canned/preserved meat and meat products, fish/seafood, vegetables, tomatoes, beans, fruit, ready meals, soup, pasta, and other canned/preserved foods.

The Frozen Processed Food category includes, but is not limited to frozen processed red meat, processed poultry, processed fish/seafood, processed vegetables, meat substitutes, processed potatoes, bakery products, desserts, ready meals, pizza, soup, noodles, and other frozen food.

The Dried Processed Food category includes, but is not limited to rice, dessert mixes, dried ready meals, dehydrated soup, instant soup, dried pasta, plain noodles, and instant noodles. The Chill Processed Food category includes, but is not limited to chilled processed meats, processed fish/seafood products, lunch kits, fresh cut fruits, ready meals, pizza, prepared salads, soup, fresh pasta and noodles.

The Sauces, Dressings and Condiments category includes, but is not limited to tomato pastes and purees, bouillon/stock cubes, herbs and spices, monosodium glutamate (MSG), table sauces, soy based sauces, pasta sauces, wet/cooking sauces, dry sauces/powder mixes, ketchup, mayonnaise, mustard, salad dressings, vinaigrettes, dips, pickled products, and other sauces, dressings and condiments.

The Baby Food category includes, but is not limited to milk- or soybean-based formula; and prepared, dried and other baby food.

The Spreads category includes, but is not limited to jams and preserves, honey, chocolate spreads, nut based spreads, and yeast based spreads.

The Dairy Product category generally refers to edible product produced from mammal's milk. Examples of dairy product include, but are not limited to drinking milk products, cheese, yoghurt and sour milk drinks, and other dairy products.

Additional examples for flavored products, particularly food and beverage products or formulations, are provided as follows. Exemplary ingestible compositions include one or more confectioneries, chocolate confectionery, tablets, countlines, bagged selflines/softlines, boxed assortments, standard boxed assortments, twist wrapped miniatures, seasonal chocolate, chocolate with toys, alfajores, other chocolate confectionery, mints, standard mints, power mints, boiled sweets, pastilles, gums, jellies and chews, toffees, caramels and nougat, medicated confectionery, lollipops, liquorice, other sugar confectionery, bread, packaged/industrial bread, unpackaged/artisanal bread, pastries, cakes, packaged/industrial cakes, unpackaged/artisanal cakes, cookies, chocolate coated biscuits, sandwich biscuits, filled biscuits, savory biscuits and crackers, bread substitutes, breakfast cereals, rte cereals, family breakfast cereals, flakes, muesli, other cereals, children's breakfast cereals, hot cereals, ice cream, impulse ice cream, single portion dairy ice cream, single portion water ice cream, multi-pack dairy ice cream, multi-pack water ice cream, take-home ice cream, take-home dairy ice cream, ice cream desserts, bulk ice cream, take-home water ice cream, frozen yoghurt, artisanal ice cream, dairy products, milk, fresh/pasteurized milk, full fat fresh/pasteurized milk, semi skimmed fresh/pasteurized milk, long-life/uht milk, full fat long life/uht milk, semi skimmed long life/uht milk, fat-free long life/uht milk, goat milk, condensed/evaporated milk, plain condensed/evaporated milk, flavored, functional and other condensed milk, flavored milk drinks, dairy only flavored milk drinks, flavored milk drinks with fruit juice, soy milk, sour milk drinks, fermented dairy drinks, coffee whiteners, powder milk, flavored powder milk drinks, cream, cheese, processed cheese, spreadable processed cheese, unspreadable processed cheese, unprocessed cheese, spreadable unprocessed cheese, hard cheese, packaged hard cheese, unpackaged hard cheese, yoghurt, plain/natural yoghurt, flavored yoghurt, fruited yoghurt, probiotic yoghurt, drinking yoghurt, regular drinking yoghurt, probiotic drinking yoghurt, chilled and shelf-stable desserts, dairy-based desserts, soy-based desserts, chilled snacks, fromage frais and quark, plain fromage frais and quark, flavored fromage frais and quark, savory fromage frais and quark, sweet and savory snacks, fruit snacks, chips/crisps, extruded snacks, tortilla/corn chips, popcorn, pretzels, nuts, other sweet and savory snacks, snack bars, granola bars, breakfast bars, energy bars, fruit bars, other snack bars, meal replacement products, slimming products, convalescence drinks, ready meals, canned ready meals, frozen ready meals, dried ready meals, chilled ready meals, dinner mixes, frozen pizza, chilled pizza, soup, canned soup, dehydrated soup, instant soup, chilled soup, hot soup, frozen soup, pasta, canned pasta, dried pasta, chilled/fresh pasta, noodles, plain noodles, instant noodles, cups/bowl instant noodles, pouch instant noodles, chilled noodles, snack noodles, canned food, canned meat and meat products, canned fish/seafood, canned vegetables, canned tomatoes, canned beans, canned fruit, canned ready meals, canned soup, canned pasta, other canned foods, frozen food, frozen processed red meat, frozen processed poultry, frozen processed fish/seafood, frozen processed vegetables, frozen meat substitutes, frozen potatoes, oven baked potato chips, other oven baked potato products, non-oven frozen potatoes, frozen bakery products, frozen desserts, frozen ready meals, frozen pizza, frozen soup, frozen noodles, other frozen food, dried food, dessert mixes, dried ready meals, dehydrated soup, instant soup, dried pasta, plain noodles, instant noodles, cups/bowl instant noodles, pouch instant noodles, chilled food, chilled processed meats, chilled fish/seafood products, chilled processed fish, chilled coated fish, chilled smoked fish, chilled lunch kit, chilled ready meals, chilled pizza, chilled soup, chilled/fresh pasta, chilled noodles, oils and fats, olive oil, vegetable and seed oil, cooking fats, butter, margarine, spreadable oils and fats, functional spreadable oils and fats, sauces, dressings and condiments, tomato pastes and purees, bouillon/stock cubes, stock cubes, gravy granules, liquid stocks and fonds, herbs and spices, fermented sauces, soy based sauces, pasta sauces, wet sauces, dry sauces/powder mixes, ketchup, mayonnaise, regular mayonnaise, mustard, salad dressings, regular salad dressings, low fat salad dressings, vinaigrettes, dips, pickled products, other sauces, dressings and condiments, baby food, milk formula, standard milk formula, follow-on milk formula, toddler milk formula, hypoallergenic milk formula, prepared baby food, dried baby food, other baby food, spreads, jams and preserves, honey, chocolate spreads, nut-based spreads, and yeast-based spreads. Exemplary ingestible compositions also include confectioneries, bakery products, ice creams, dairy products, sweet and savory snacks, snack bars, meal replacement products, ready meals, soups, pastas, noodles, canned foods, frozen foods, dried foods, chilled foods, oils and fats, baby foods, or spreads or a mixture thereof. Exemplary ingestible compositions also include breakfast cereals, sweet beverages or solid or liquid concentrate compositions for preparing beverages, ideally so as to enable the reduction in concentration of previously known saccharide sweeteners, or artificial sweeteners.

Some embodiments provide a chewable composition that may or may not be intended to be swallowed. In some embodiments, the chewable composition may be gum, chewing gum, sugarized gum, sugar-free gum, functional gum, bubble gum including compounds as disclosed and described herein, individually or in combination.

Typically at least a sweet receptor modulating amount, a sweet receptor ligand modulating amount, a sweet flavor modulating amount, a sweet flavoring agent amount, a sweet flavor enhancing amount, or a therapeutically effective amount of one or more of the present compounds will be added to the ingestible composition, optionally in the presence of sweeteners so that the sweet flavor modified ingestible composition has an increased sweet taste as compared to the ingestible composition prepared without the compounds of the present invention, as judged by human beings or animals in general, or in the case of formulations testing, as judged by a majority of a panel of at least eight human taste testers, via procedures commonly known in the field.

In some embodiments, compounds as disclosed and described herein, individually or in combination, modulate the sweet taste or other taste properties of other natural or synthetic sweet tastants, and ingestible compositions made therefrom. In one embodiment, the compounds as disclosed and described herein, individually or in combination, may be used or provided in its ligand enhancing concentration(s). For example, the compounds as disclosed and described herein, individually or in combination, may be present in an amount of from 0.001 ppm to 100 ppm, or narrower alternative ranges from 0.1 ppm to 50 ppm, from 0.01 ppm to 40 ppm, from 0.05 ppm to 30 ppm, from 0.01 ppm to 25 ppm, or from 0.1 ppm to 30 ppm, or from 0.1 ppm to 25 ppm, or from 1 ppm to 30 ppm, or from 1 ppm to 25 ppm.

In some embodiments, flavor-modifying compounds as disclosed and described herein, individually or in combination, may be provided in a flavoring concentrate formulation, e.g., suitable for subsequent processing to produce a ready-to-use (i.e., ready-to-serve) product. By "a flavoring concentrate formulation", it is meant a formulation which should be reconstituted with one or more diluting medium to become a ready-to-use composition. The term "ready-to-use composition" is used herein interchangeably with "ingestible composition", which denotes any substance that, either alone or together with another substance, can be taken by mouth whether intended for consumption or not. In one embodiment, the ready-to-use composition includes a composition that can be directly consumed by a human or animal. The flavoring concentrate formulation is typically used by mixing with or diluted by one or more diluting medium, e.g., any consumable or ingestible ingredient or product, to impart or modify one or more flavors to the diluting medium. Such a use process is often referred to as reconstitution. The reconstitution can be conducted in a household setting or an industrial setting. For example, a frozen fruit juice concentrate can be reconstituted with water or other aqueous medium by a consumer in a kitchen to obtain the ready-to-use fruit juice beverage. In another example, a soft drink syrup concentrate can be reconstituted with water or other aqueous medium by a manufacturer in large industrial scales to produce the ready-to-use soft drinks. Since the flavoring concentrate formulation has the flavoring agent or flavor modifying agent in a concentration higher than the ready-to-use composition, the flavoring concentrate formulation is typically not suitable for being consumed directly without reconstitution. There are many benefits of using and producing a flavoring concentrate formulation. For example, one benefit is the reduction in weight and volume for transportation as the flavoring concentrate formulation can be reconstituted at the time of usage by the addition of suitable solvent, solid or liquid.

The flavored products set forth according to any of the foregoing embodiments, also include, in certain embodiments, one or more additional flavor-modifying compounds, such as compounds that enhance sweetness (e.g., hesperetin, naringenin, glucosylated steviol glycosides, etc.), compounds that block bitterness, compounds that enhance umami, compounds that reduce sourness, compounds that enhance saltiness, compounds that enhance a cooling effect, or any combinations of the foregoing.

In certain embodiments of any aspects and embodiments set forth herein that refer to a sweetening or flavoring concentrate, the sweetening or flavoring concentrate is a non-naturally-occurring product, such as a composition specifically manufactured for the production of a flavored product, such as food or beverage product.

In one embodiment, the flavoring concentrate formulation comprises i) compounds as disclosed and described herein, individually or in combination; ii) a carrier; and iii) optionally at least one adjuvant. The term "carrier" denotes a usually inactive accessory substance, such as solvents, binders, or other inert medium, which is used in combination with the present compound and one or more optional adjuvants to form the formulation. For example, water or starch can be a carrier for a flavoring concentrate formulation. In some embodiments, the carrier is the same as the diluting medium for reconstituting the flavoring concentrate formulation; and in other embodiments, the carrier is different from the diluting medium. The term "carrier" as used herein includes, but is not limited to, ingestibly acceptable carrier.

The term "adjuvant" denotes an additive which supplements, stabilizes, maintains, or enhances the intended function or effectiveness of the active ingredient, such as the compound of the present invention. In one embodiment, the at least one adjuvant comprises one or more flavoring agents. The flavoring agent may be of any flavor known to one skilled in the art or consumers, such as the flavor of chocolate, coffee, tea, mocha, French vanilla, peanut butter, chai, or combinations thereof. In another embodiment, the at least one adjuvant comprises one or more sweeteners. The one or more sweeteners can be any of the sweeteners described in this application. In another embodiment, the at least one adjuvant comprises one or more ingredients selected from the group consisting of a emulsifier, a stabilizer, an antimicrobial preservative, an antioxidant, vitamins, minerals, fats, starches, protein concentrates and isolates, salts, and combinations thereof. Examples of emulsifiers, stabilizers, antimicrobial preservatives, antioxidants, vitamins, minerals, fats, starches, protein concentrates and isolates, and salts are described in U.S. Pat. No. 6,468,576, the content of which is hereby incorporated by reference in its entirety for all purposes.

In one embodiment, the present flavoring concentrate formulation can be in a form selected from the group consisting of liquid including solution and suspension, solid, foamy material, paste, gel, cream, and a combination thereof, such as a liquid containing certain amount of solid contents. In one embodiment, the flavoring concentrate formulation is in form of a liquid including aqueous-based and nonaqueous-based. In some embodiments, the present flavoring concentrate formulation can be carbonated or non-carbonated.

The flavoring concentrate formulation may further comprise a freezing point depressant, nucleating agent, or both as the at least one adjuvant. The freezing point depressant is an ingestibly acceptable compound or agent which can depress the freezing point of a liquid or solvent to which the compound or agent is added. That is, a liquid or solution containing the freezing point depressant has a lower freezing point than the liquid or solvent without the freezing point depressant. In addition to depress the onset freezing point, the freezing point depressant may also lower the water activity of the flavoring concentrate formulation. The examples of the freezing point depressant include, but are not limited to, carbohydrates, oils, ethyl alcohol, polyol, e.g., glycerol, and combinations thereof. The nucleating agent denotes an ingestibly acceptable compound or agent which is able to facilitate nucleation. The presence of nucleating agent in the flavoring concentrate formulation can improve the mouthfeel of the frozen Blushes of a frozen slush and to help maintain the physical properties and performance of the slush at freezing temperatures by increasing the number of desirable ice crystallization centers. Examples of nucleating agents include, but are not limited to, calcium silicate, calcium carbonate, titanium dioxide, and combinations thereof.

In one embodiment, the flavoring concentrate formulation is formulated to have a low water activity for extended shelf life. Water activity is the ratio of the vapor pressure of water in a formulation to the vapor pressure of pure water at the same temperature. In one embodiment, the flavoring concentrate formulation has a water activity of less than about 0.85. In another embodiment, the flavoring concentrate formulation has a water activity of less than about 0.80. In another embodiment, the flavoring concentrate formulation has a water activity of less than about 0.75.

In one embodiment, the flavoring concentrate formulation has the present compound in a concentration that is at least 2 times of the concentration of the compound in a ready-to-use composition. In one embodiment, the flavoring concentrate formulation has the present compound in a concentration that is at least 5 times of the concentration of the compound in a ready-to-use composition. In one embodiment, the flavoring concentrate formulation has the present compound in a concentration that is at least 10 times of the concentration of the compound in a ready-to-use composition. In one embodiment, the flavoring concentrate formulation has the present compound in a concentration that is at least 15 times of the concentration of the compound in a ready-to-use composition. In one embodiment, the flavoring concentrate formulation has the present compound in a concentration that is at least 20 times of the concentration of the compound in a ready-to-use composition. In one embodiment, the flavoring concentrate formulation has the present compound in a concentration that is at least 30 times of the concentration of the compound in a ready-to-use composition. In one embodiment, the flavoring concentrate formulation has the present compound in a concentration that is at least 40 times of the concentration of the compound in a ready-to-use composition. In one embodiment, the flavoring concentrate formulation has the present compound in a concentration that is at least 50 times of the concentration of the compound in a ready-to-use composition. In one embodiment, the flavoring concentrate formulation has the present compound in a concentration that is at least 60 times of the concentration of the compound in a ready-to-use composition. In one embodiment, the flavoring concentrate formulation has the present compound in a concentration that is up to 100 times of the concentration of the compound in a ready-to-use composition.

The sweetening or flavoring concentrates set forth according to any of the foregoing embodiments, also include, in certain embodiments, one or more additional flavor-modifying compounds, such as compounds that enhance sweetness (e.g., hesperetin, naringenin, glucosylated steviol glycosides, etc.), compounds that block bitterness (e.g., eriodictyol, homoeriodictyol, sterubin, and salts or glycoside derivatives thereof, as well as vanillyl lignans, e.g., matairesinol and other compounds set forth in PCT Publication No. WO 2012/146584), compounds that enhance umami (e.g., rubemamine, rubescenamine, (E)-3-(3,4-dimethoxyphenyl)-N-(4-methoxyphenethyl)acrylamide, and the like), compounds that reduce sourness and/or licorice taste, compounds that enhance saltiness, compounds that enhance a cooling effect, or any combinations of the foregoing.

### Methods of Preparation

The flavor-modifying compounds disclosed herein may be synthesized by methods described below, or by modification of these methods. Ways of modifying the methodology include, among others, temperature, solvent, reagents etc., known to those skilled in the art. In general, during any of the processes for preparation of the compounds disclosed herein, it may be necessary or desirable to protect sensitive or reactive groups on any of the molecules concerned.

### Non-Animal Protein Materials and Products Made Therefrom

Products intended to replace or substitute meat or dairy products often rely on various non-animal-based materials, such as starches and proteins derived from plants, algae, fungi, or combinations thereof, to simulate the texture and flavor of meat or dairy. Non-limiting examples of non-animal-based proteins are plant proteins, such as soy proteins, pea proteins, bean proteins, grain proteins, and the like. Due to compositional differences between such plant-based materials and animal-derived materials, such as a lack of glutamate-containing proteins and glutathione, these products can lack the umami and/or kokumi taste that consumers traditionally associate with meat or dairy products.

Thus, in certain aspects, the disclosure provides a flavored product comprising a plant-based material (such as a plant-based starch, a plant-based protein, or a combination thereof) and the flavor-modifying compounds disclosed herein. In some further embodiments, the flavored product can include any features of combination of features set forth above for ingestible compositions that contain the flavor-modifying compounds disclosed herein. In some embodiments, the flavored product is a beverage, such as soy milk, almond milk, rice milk, oat milk, a protein drink, a meal-replacement drink, or other like product. In some other embodiments, the flavored product is a meat-replacement product, such as a plant-based chicken product (such as a plant-based chicken nugget), a plant-based beef product (such as a plant-based burger), and the like. In some other embodiments, the flavored product is a protein powder, a meal-replacement powder, a plant-based creamer for coffee or tea, and the like. In certain further embodiments, any such products contain additional ingredients, and have additional features, as are typically used in the preparation and/or manufacture of such products. For example, the flavor-modifying compounds disclosed herein, may be combined with other flavors and taste modifiers, and may even be encapsulated in certain materials, according to known technologies in the relevant art. Suitable concentrations of the flavor-modifying compounds disclosed herein, are set forth above.

In some embodiments, the flavored products comprise one or more plant-based proteins, which impart a bitter taste that is at least partially reduced by the use of the flavor-modifying compounds disclosed herein in the product. Such plant-based proteins include, but are not limited to, pea protein, soy protein, canola (rapeseed) protein, barley protein, chickpea protein, mycoproteins, algal proteins, fava protein, sunflower protein, wheat protein,

In some alternative embodiments analogous to the above embodiments, algal or fungal proteins or starches are used instead, oat protein, potato protein, and the like. In some embodiments, these flavored products also include fiber to provide texture to the product. Fibers suitable for use include, but are not limited to, psyllium fiber, pea fiber, potato fiber, curdlan, soluble corn fiber (dextran and/or maltodextrin), citrus fiber, barley fiber, and combinations thereof. In such products, the flavor-modifying compounds disclosed herein can be introduced in any suitable way. In some embodiments, the flavor-modifying compounds disclosed herein is incorporated into a flavoring emulsion, such as a water-in-oil emulsion, along with other flavor-imparting ingredients.

### Non-Meat Protein Materials and Products Made Therefrom

Certain non-meat animal proteins, such as dairy proteins and proteins from bone broth, are commonly used in food products, and are also sold as the primary ingredient in certain protein powders. Such proteins can impart bitter flavors that consumers may not desire. This is especially true for protein isolates, such as protein isolates of whey protein, collagen protein, casein proteins, and the like. Thus, the present disclosure provides ingestible compositions that include non-meat animal proteins and the flavor-modifying compounds disclosed herein. The flavor-modifying compounds disclosed herein can be present in any suitable combination, according to the embodiments set forth in the preceding sections of the present disclosure. In some embodiments, the non-meat animal protein is a bone protein, such as a collagen protein derived from the bones of an animal, such as a cow, pig, donkey, horse, chicken, duck, goat, goose, rabbit, lamb, sheep, buffalo, ostrich, camel, and the like. In some embodiments, the non-meat animal protein is a milk protein, such as a whey protein, a casein protein, or any combination thereof. The milk can be the milk of any suitable animal, such as a cow, donkey, horse, sheep, buffalo, camel, and the like.

The flavor-modifying compounds disclosed herein, can also be included in certain food or beverage products that include animal milk or materials derived from animal milk. Such products include cheeses, cheese spreads, yogurt, kefir, milk, processed dairy products, cottage cheese, sour cream, butter, and the like.

### Blocking Bitterness in Pharmaceutical APIs

Many drug compounds impart a bitter taste, which therefore limits the ways in which they can be formulated and administered. Therefore, in certain aspects, the disclosure provides a pharmaceutical composition comprising a bitter-tasting pharmaceutical active ingredient, and the flavor-modifying compounds disclosed herein. Such pharmaceutical compositions can be in any suitable form for oral administration, such as tablets, lozenges, capsules, powders, liquid solutions, liquid suspensions, and the like. Such pharmaceutical compositions can include any suitable pharmaceutical excipients, binders, and the like, such as those set forth in *Remington's Pharmaceutical Sciences*. In some embodiments, the bitter-tasting pharmaceutical active ingredient is an ion channel inhibitor, such as a proton channel inhibitor. Other examples of bitter-tasting APIs whose bitterness is reduced by the flavor-modifying compounds disclosed herein, include, but are not limited to, atropine, brinzolamide, chloramphenicol, chloroquine, clindamycin, dexamethasone, digoxin, diltiazem, diphenhydramine, docusate, dorzolamide, doxepin, doxylamine, enalapril, erythromycin, esomeprazole, famotidine, gabapentin, ginkgolide A, guaifenesin, L-histidine, lomefloxacin, methylprednisolone, ofloxacin, oleuropein, oxyphenonium, pirenzepine, prednisone, ranitidine, trapidil, trimethoprim, and cetirizine.

### Use in Oral Care Products

Oral care products often contain ingredients that impart astringent or bitter off tastes. Such ingredients include menthol, menthol analogues, mint extracts, sodium bicarbonate, alkali metal salts of peroxymonosulfate (potassium peroxymonosulfate), cetylpyridinium chloride, lauramidopropyl betaine, cocamidopropyl betaine, arginine, hydrogen peroxide, chlorhexidine gluconate, zinc phosphate, zinc chloride, zinc citrate, potassium nitrate, pentasodium triphosphate, tetrasodium pyrophosphate, stannous fluoride, thymol, methyl salicylate, eucalyptol, or any combination thereof. Suitable oral care products include toothpaste, mouthwashes, whitening agents, dentifrices, and the like. Thus, such oral care products can, in certain embodiments, comprise the flavor-modifying compounds disclosed herein to block the bitterness or off tastes of such compounds commonly used.

### EXAMPLES

To further illustrate this invention, the following examples are included. The examples should not, of course, be construed as specifically limiting the invention. Variations of these examples within the scope of the claims are within the purview of one skilled in the art and are considered to fall within the scope of the invention as described, and claimed herein. The reader will recognize that the skilled artisan, armed with the present disclosure, and skill in the art is able to prepare and use the invention without exhaustive examples.

### Example 1 - Disodium Salt of Naringenin (~10% Aqueous Solution)

Naringenin (500 mg, 1.84 mmol) was suspended in water (2mL) and while stirring a solution of NaOH (2 equiv.) in water (3 mL) was added dropwise. After addition was complete, the reaction was bubbled with nitrogen and stirring continued until the solution became homogeneous. The solution could be stored for several days at room temperature under nitrogen without any significant change. ¹H NMR (400 MHz, DMSO-d₆) δ 12.67 (s, 1H), 6.84 - 6.77 (m, 2H), 6.07 (d, J = 8.0 Hz, 2H), 4.98 (s, 1H), 4.82 (dd, J = 12.7, 2.9 Hz, 1H), 2.85 (dd, J = 16.7, 13.0 Hz, 1H), 2.16 (dd, J = 16.8, 2.8 Hz, 1H). MS 273 (M + 3H - 2Na)⁺.

### Example 2: Dipotassium Salt of Naringenin (~10% Aqueous Solution)

Naringenin (500 mg, 1.84 mmol) was suspended in water (2mL) and while stirring a solution of KOH (2 equiv.) in water (3mL) was added dropwise. After addition was complete, the reaction was bubbled with nitrogen and stirring continued until the solution became homogeneous. The solution could be stored for several days at room temperature under nitrogen without any significant change. ¹H NMR (400 MHz, DMSO-d6) δ 12.67 (s, 1H), 6.89 (d, J = 8.4 Hz, 2H), 6.23 (d, J = 8.0 Hz, 2H), 5.02 - 4.96 (m, 2H), 4.86 (dd, J = 12.7, 2.8 Hz, 1H), 2.85 (dd, J = 16.8, 12.8 Hz, 1H), 2.19 (dd, J = 16.8, 2.9 Hz, 1H). MS 273 (M + 3H - 2K)⁺.

### Example 3: Disodium Salt of Phloterin (~10% Aqueous Solution)

Phloterin (500 mg, 1.82 mmol) was suspended in water (2 mL) and while stirring a solution of NaOH (2 equiv.) in water (3 mL) was added dropwise. After addition was complete, the reaction was bubbled with nitrogen and stirring continued until the solution became homogeneous. The solution could be stored for several days at room temperature under nitrogen without any significant change. ¹H NMR (400 MHz, DMSO-d6) δ 6.87 (d, J = 7.9 Hz, 2H), 6.59 (s, 2H), 4.94 (s, 1H), 4.70 (s, 1H), 2.64 - 2.55 (m, 4H). MS 275 (M + 3H - 2Na)⁺.

### Example 4: Dipotassium Salt of Phloterin (~10% Aqueous Solution)

Phloterin (500mg, 1.82 mmol) was suspended in water (2mL) and while stirring a solution of KOH (2 equiv.) in water (3mL) was added dropwise. After addition was complete, the reaction was bubbled with nitrogen and stirring continued until the solution became homogeneous. The solution could be stored for several days at room temperature under nitrogen without any significant change. ¹H NMR (400 MHz, DMSO-*d*₆) δ 6.88 (d, *J* = 7.9 Hz, 2H), 6.62 (s, 1H), 4.95 (s, 1H), 4.71 (s, 1H), 2.64 - 2.52 (m, 4H). MS 275 (M + 3H - 2K)⁺.
The present invention is also described by the following items:
1. A flavor-modifying compound, which is a di-alkali metal salt or a tri-alkali metal salt of a compound of formula (I): 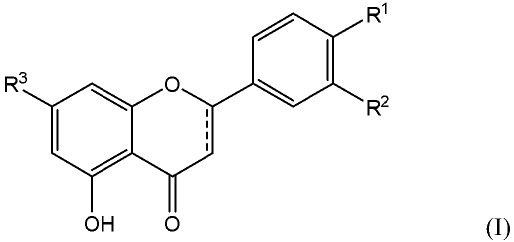 wherein R¹, R², and R³ are independently a hydrogen atom, -OH, or -OCH₃, and at least one of R¹, R², and R³ is -OH; wherein the alkali metal of the di-alkali metal salt and tri-alkali metal salt is sodium, potassium, or a combination thereof; and where the dotted line represents an optional carbon-carbon double bond.
2. The flavor-modifying compound of item 1, which is a disodium or a dipotassium salt of a compound of formula (I).
3. The flavor-modifying compound of item 1 or 2, wherein the compound of formula (I) is naringenin, wherein the naringenin is in its pure (S) form.
4. The flavor-modifying compound of item 1 or 2, wherein the compound of formula (I) is naringenin, wherein the naringenin is racemic mixture of its (R) form and its (S) form.
5. A flavor-modifying compound, which is a di-alkali metal salt or a tri-alkali metal salt of a compound of formula (II): 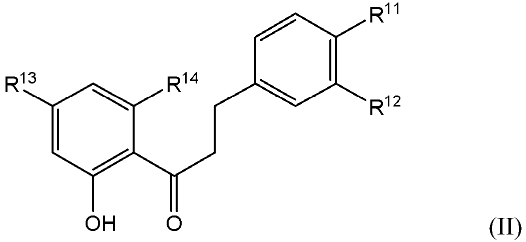 wherein R¹¹, R¹², R¹³, and R¹⁴ are independently a hydrogen atom, -OH, or -OCH₃, and at least one of R¹¹, R¹², R¹³, and R¹⁴ is -OH; and wherein the alkali metal of the di-alkali metal salt and tri-alkali metal salt is sodium, potassium, or a combination thereof.
6. The flavor-modifying compound of item 5, which is a disodium or a dipotassium salt of a compound of formula (II).
7. The flavor-modifying compound of item 5 or 6, wherein R¹¹ is -OH or -OCH₃, R¹² is a hydrogen atom or -OH, R¹³ is -OH, and R¹⁴ is a hydrogen atom or -OH.
8. The flavor-modifying compound of item 7, wherein the compound of formula (II) is phloretin.
9. A flavor-modifying compound, which is a di-alkali metal salt or a tri-alkali metal salt of a compound of formula (III): 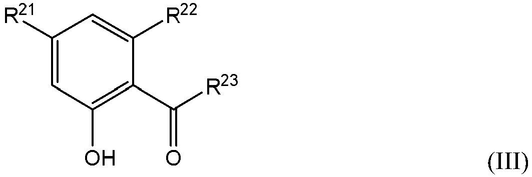 wherein R²¹ and R²² are independently a hydrogen atom, -OH, or -OCH₃, and at least one of R²¹ and R²² is -OH; R²³ is C₁₋₆ alkyl or -CH₂-R²⁴; and R²⁴ is phenyl optionally substituted from 1 to three times by -OH, -OCH₃, or a combination thereof; and wherein the alkali metal of the di-alkali metal salt and tri-alkali metal salt is sodium, potassium, or a combination thereof.
10. The flavor-modifying compound of item 9, which is a disodium or a dipotassium salt of a compound of formula (III).
11. Use of a flavor-modifying compound of any one of items 1 to 10 to enhance a sweet taste of an ingestible composition.
12. Use of a flavor-modifying compound of any one of items 1 to 10 to reduce a bitter taste of an ingestible composition.
13. Use of a flavor-modifying compound of any one of items 1 to 10 to reduce a caloric content of an ingestible composition.
14. The use of any one of items 11 to 13, wherein the ingestible comprises a sweetener, a bitter tastant, or a combination thereof.
15. An ingestible composition comprising a flavor-modifying compound of any one of items 1 to 10 and a sweetener, wherein the sweetener is a steviol glycoside, a mogroside, or a combination thereof.

## Claims

1. A flavor-modifying compound, which is a di-alkali metal salt or a tri-alkali metal salt of a compound of formula (II): 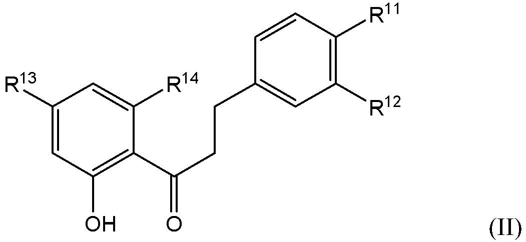 wherein R¹¹, R¹², R¹³, and R¹⁴ are independently a hydrogen atom, -OH, or -OCH₃, and at least one of R¹¹, R¹², R¹³, and R¹⁴ is -OH; and wherein the alkali metal of the di-alkali metal salt and tri-alkali metal salt is sodium, potassium, or a combination thereof.

2. The flavor-modifying compound of claim 1, which is a disodium or a dipotassium salt of a compound of formula (II).

3. The flavor-modifying compound of claim 1 or 2, wherein R¹¹ is -OH or -OCH₃, R¹² is a hydrogen atom or -OH, R¹³ is -OH, and R¹⁴ is a hydrogen atom or -OH.

4. The flavor-modifying compound of claim 3, wherein the compound of formula (II) is phloretin.

5. Use of a flavor-modifying compound of any one of claims 1 to 4 to enhance a sweet taste of an ingestible composition.

6. Use of a flavor-modifying compound of any one of claims 1 to 4 to reduce a bitter taste of an ingestible composition.

7. Use of a flavor-modifying compound of any one of claims 1 to 4 to reduce a caloric content of an ingestible composition.

8. The use of any one of claims 5 to 7, wherein the ingestible comprises a sweetener, a bitter tastant, or a combination thereof.

9. An ingestible composition comprising a flavor-modifying compound of any one of claims 1 to 4 and a sweetener, wherein the sweetener is a steviol glycoside, a mogroside, or a combination thereof.
